(19) Europäisches Patentamt
European Patent Office
Office européen des brevets

(11) EP 4 327 833 A1

(12) **EUROPEAN PATENT APPLICATION**
published in accordance with Art. 153(4) EPC

(43) Date of publication:
**28.02.2024 Bulletin 2024/09**

(21) Application number: **22791778.8**

(22) Date of filing: **21.04.2022**

(51) International Patent Classification (IPC):
***A61K 51/10*** *(2006.01)* ***A61K 31/198*** *(2006.01)*
***A61K 31/341*** *(2006.01)* ***A61K 31/585*** *(2006.01)*
***A61K 33/24*** *(2019.01)* ***A61K 39/395*** *(2006.01)*
***A61K 45/00*** *(2006.01)* ***A61K 47/22*** *(2006.01)*
***A61K 47/69*** *(2017.01)* ***A61P 13/12*** *(2006.01)*
***A61P 35/00*** *(2006.01)* ***A61P 39/04*** *(2006.01)*
***A61P 43/00*** *(2006.01)* ***C07K 16/18*** *(2006.01)*
***C07K 16/46*** *(2006.01)*

(52) Cooperative Patent Classification (CPC):
**A61K 31/198; A61K 31/341; A61K 31/585; A61K 33/24; A61K 39/395; A61K 45/00; A61K 47/22; A61K 47/69; A61K 51/10; A61P 13/12; A61P 35/00; A61P 39/04; A61P 43/00; C07K 16/18; C07K 16/46**

(86) International application number:
**PCT/JP2022/018414**

(87) International publication number:
**WO 2022/225007 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.04.2021 JP 2021072206**

(71) Applicants:
- **Nihon Medi-Physics Co., Ltd.**
  **Tokyo 136-0075 (JP)**
- **Sumitomo Pharma Co., Ltd.**
  **Osaka 541-0045 (JP)**

(72) Inventors:
- **KOMOTO, Shota**
  **Tokyo 136-0075 (JP)**
- **YAMADA, Naoaki**
  **Tokyo 136-0075 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **RADIOACTIVE ANTITUMOR AGENT**

(57) The present invention aims to provide a Actinium-225-labeled anti-MUC5AC humanized antibody that is superior in the specificity for mucin subtype 5AC (MUC5AC) and accumulation in tumor, and shows reduced renal toxicity. The present invention relates to a conjugate of a chelating agent chelated with Actinium-225 and an antibody, wherein the antibody is a humanized antibody that specifically binds to mucin subtype 5AC and has a heavy chain variable region consisting of the amino acid sequence shown in any of SEQ ID NOs: 1 to 4, and a light chain variable region consisting of the amino acid sequence shown in any of SEQ ID NOs: 5 to 8.

EP 4 327 833 A1

[Fig. 1]

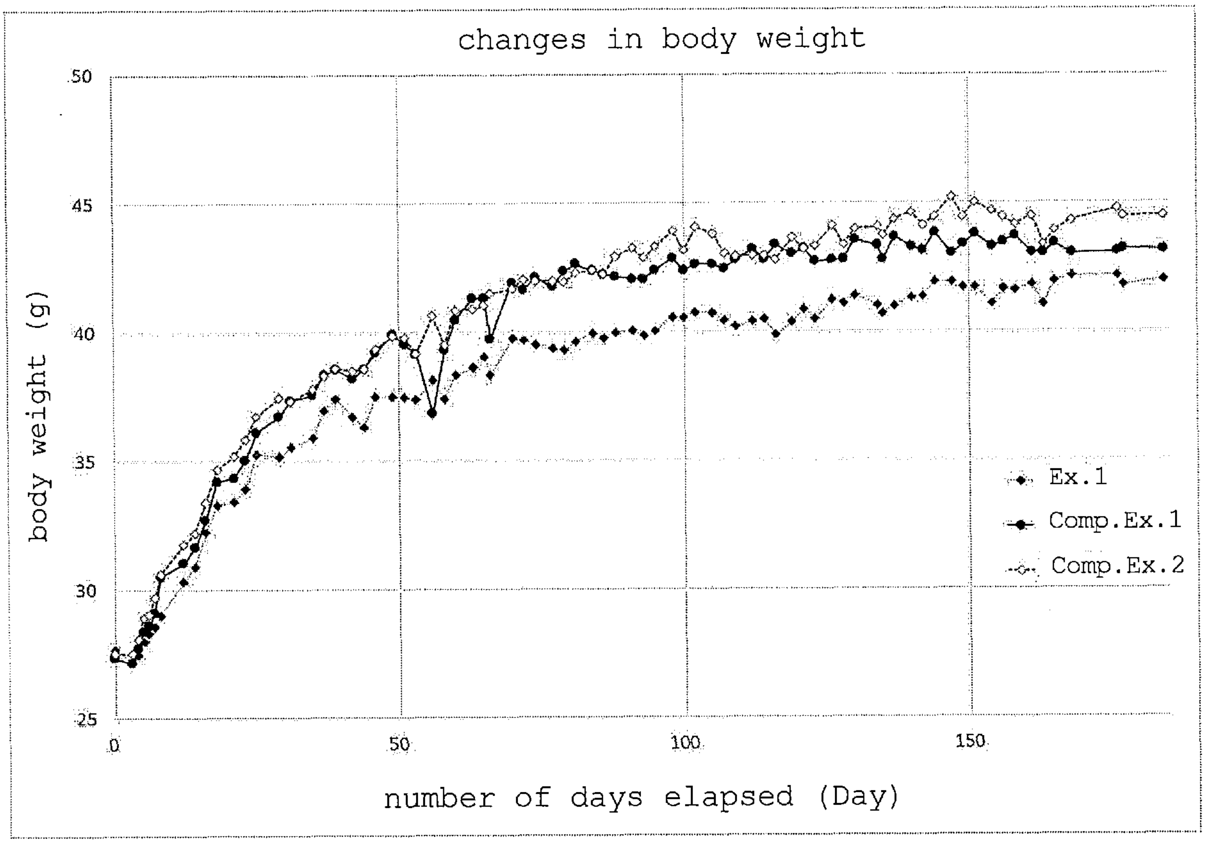
changes in body weight
body weight (g)
50
45
40
35
30
25
0
50
100
150
number of days elapsed (Day)
Ex.1
Comp.Ex.1
Comp.Ex.2

## Description

[Technical Field]

The present invention relates to a radioactive antitumor agent. More particularly, the present invention relates to a radioactive antitumor agent to be used in combination with a drug for reducing renal toxicity, comprising, as an active ingredient, a conjugate of a chelating agent chelated with a radioactive nuclide $^{225}$Ac (Actinium-225) and a humanized antibody that specifically binds to mucin subtype 5AC, and the like.

[Background Art]

Mucin is the main component of mucus secreted from epithelial cell and the like of animal and is a glycoprotein containing a large amount of sugar with a molecular weight of 1 - 10 million. Mucin includes secretory mucin produced by epithelial cell and the like and membrane-bound mucin that has a hydrophobic transmembrane site and exists while being bound to the cell membrane. The core proteins of mucin are collectively called MUC, and it is known that there are at least 20 types of genes encoding core proteins. One of them, mucin subtype 5AC (MUC5AC), belongs to secretory mucin.

MUC5AC is expressed in the stomach and trachea in normal tissues, and overexpression in pancreatic cancer has been reported. Overexpression has also been reported in thyroid cancer, liver cancer, colorectal cancer, gastric cancer, urothelial cancer, breast cancer, cervical cancer, ovarian cancer, endometrial cancer, and bile duct cancer. As antibodies to MUC5AC, a mouse antibody (Non-Patent Literature 1) prepared using, as an antigen, a pancreatic cancer mucin fraction purified from xenograft of human pancreatic cancer cell line SW1990, and chimeric antibodies (Patent Literatures 1, 2, Non-Patent Literatures 2, 3) and humanized antibody (Patent Literatures 3, 4) produced based thereon have been reported.

An antibody is used as a reagent for detecting a target molecule, a diagnostic agent, or a pharmaceutical product for treating a disease by utilizing the specificity of the antibody for a target molecule. To further improve the detection performance and therapeutic effect, studies on antibodies bound to radionuclides and drugs are underway. Non-Patent Literature 1 has reported radioimmunotherapy of pancreatic carcinoma model mice using mouse antibody labeled with $^{131}$I which is a β-ray emitting nuclide (β nuclide). Non-Patent Literature 3 has reported SPECT imaging of pancreatic cancer patients using a chimeric antibody labeled with $^{111}$In which is a γ-ray emitting nuclide (γ nuclide). Patent Literatures 3 and 4 describe particular MUC5AC-specific humanized antibodies and also describe that those labeled with $^{90}$Y, $^{111}$In, etc. are included. However, they only show the ADCC (antibody-dependent cellular cytotoxicity) activity of the MUC5AC-specific humanized antibody in the Examples.

Patent Literature 5 and Non-Patent Literature 4 describe an antibody labeled with $^{225}$Ac, which is a nuclide that emits α-rays (α nuclide) ($^{225}$Ac-labeled antibody), and the late renal toxicity after administration of the antibody has been reported. Various drugs are used in combination to reduce renal toxicity (Non-Patent Literature 4).

Patent Literature 6 discloses a pharmaceutical composition used for renal protection, which contains a renin inhibitor having a specific structure and spironolactone and eplerenone, which are steroidal aldosterone antagonists, in combination.

[Citation List]

[Patent Literature]

Patent Literature 1: JP-A-H7-203974
Patent Literature 2: JP-A-H11-5749
Patent Literature 3: WO 2013/157102
Patent Literature 4: WO 2013/157105
Patent Literature 5: WO 2005/028021
Patent Literature 6: JP-A-2007-529459

[Non-Patent Literature]

Non-Patent Literature 1: Japanese Journal of Cancer Research, 87, 977-984, 1996

Non-Patent Literature 2: Japanese Journal of Clinical Medicine vol. 64 extra issue 1, 2006, p274-278
Non-Patent Literature 3: Japanese Journal of Cancer Research, 90, 1179-1186, 1999
Non-Patent Literature 4: Cancer Res. 2005 Jun 1; 65(11):4888-95.

[Summary of Invention]

[Technical Problem]

The present invention aims to provide, in administration of a humanized antibody labeled with a radioactive nuclide $^{225}$Ac (Actinium-225), which specifically binds to mucin subtype 5AC (hereinafter to be also referred to as a $^{225}$Ac-labeled anti-MUC5AC humanized antibody), a drug that reduces renal toxicity caused by the administration of a drug containing Actinium-225, or caused by Actinium-225 or a daughter nuclide of Actinium-225.

[Solution to Problem]

In view of the above-mentioned problem, the present inventors have conducted intensive studies and found that, by using a $^{225}$Ac-labeled anti-MUC5AC humanized antibody and a drug for reducing renal toxicity in combination, renal toxicity of a radioactive antitumor agent containing a $^{225}$Ac-labeled anti-MUC5AC humanized antibody as an active ingredient or a radioactive antitumor agent containing both a $^{225}$Ac-labeled anti-MUC5AC humanized antibody and a drug that reduces renal toxicity as active ingredients can be reduced, and completed the present invention by confirming the effect thereof.

One embodiment of the present invention provides a radioactive antitumor agent containing a $^{225}$Ac-labeled anti-MUC5AC humanized antibody as an active ingredient, which is used in combination with a drug for reducing renal toxicity.

Another embodiment of the present invention provides a radioactive antitumor agent containing a $^{225}$Ac-labeled anti-MUC5AC humanized antibody and a drug for reducing renal toxicity as active ingredients.

Another embodiment of the present invention provides a drug for reducing renal toxicity, which is used in combination with a radioactive antitumor agent containing a $^{225}$Ac-labeled anti-MUC5AC humanized antibody as an active ingredient.

Another embodiment of the present invention provides a combination drug containing a radioactive antitumor agent containing a $^{225}$Ac-labeled anti-MUC5AC humanized antibody as an active ingredient, and a drug for reducing renal toxicity.

[Brief Description of Drawings]

[Fig. 1]
Fig. 1 is a graph showing changes in the average body weight of mice 26 weeks after administration of the $^{225}$Ac-labeled anti-MUC5AC humanized antibody. The results of a group administered in combination with a compound expected to have a renal protective effect (Zn-DTPA) (Example 1), a group administered with a $^{225}$Ac-labeled anti-MUC5AC humanized antibody alone (Comparative Example 1), and a group administered with a compound expected to have a renal protective effect (Zn-DTPA) alone (Comparative Example 2) are shown.

[Fig. 2]
Fig. 2 is a graph showing changes in the average body weight of mice 26 weeks after administration of the $^{225}$Ac-labeled anti-MUC5AC humanized antibody. The results of groups administered in combination with a compound expected to have a renal protective effect (furosemide) (Example 2, Example 5), a group administered with a $^{225}$Ac-labeled anti-MUC5AC humanized antibody alone (Comparative Example 1), and a group administered with a compound expected to have a renal protective effect (furosemide) alone (Comparative Example 3) are shown.

[Fig. 3]
Fig. 3 is a graph showing changes in the average body weight of mice 26 weeks after administration of the $^{225}$Ac-labeled anti-MUC5AC humanized antibody. The results of groups administered in combination with a compound expected to have a renal protective effect (spironolactone) (Example 3, Example 6), a group administered with a $^{225}$Ac-labeled anti-MUC5AC humanized antibody alone (Comparative Example 1), and a group administered with a compound expected to have a renal protective effect (spironolactone) alone (Comparative Example 4) are shown.

[Fig. 4]
Fig. 4 is a graph showing changes in the average body weight of mice 26 weeks after administration of the $^{225}$Ac-labeled anti-MUC5AC humanized antibody. The results of groups administered in combination with a compound

expected to have a renal protective effect (eplerenone) (Example 4, Example 7), a group administered with a $^{225}$Ac-labeled anti-MUC5AC humanized antibody alone (Comparative Example 1), and a group administered with a compound expected to have a renal protective effect (eplerenone) alone (Comparative Example 5) are shown.

[Fig. 5]

Fig. 5 is a graph showing the urinary N/C ratio in mice of each administration group 16 weeks after administration of the $^{225}$Ac-labeled anti-MUC5AC humanized antibody (each group n=4 to 5). "*" indicates an administration group in which a significant difference was observed in the Dunnett test (p<0. 05) .

[Fig. 6]

Fig. 6 is a diagram showing the results of pathological evaluation of the kidneys of each administration group. Each group contained 2 to 5 mice, and the number of individuals of each grade in which the findings described in the column of findings were observed is shown. For each finding, grade "-" commonly indicates that the finding is not observed. In the degeneration/regeneration of renal tubular epithelium in the outer layer of the medulla, grade "+" indicates that degeneration of renal tubular epithelial cells is observed sporadically, grade "2+" indicates that said change is observed diffusely, and grade "3+" indicates that degeneration and regeneration of renal tubules are in a clear and active state. In basophilic tubules with inflammatory cell infiltration and fibrosis, grade "+" indicates that the finding is observed at the same level of frequency as natural development, grade "2+" indicates that the findings are observed more than natural development and are multifocal, and grade "3+" indicates that obvious changes are observed throughout the kidney.

[Fig. 7]

Fig. 7 is a diagram showing typical pathological findings of degeneration/regeneration of renal tubular epithelial cells in the outer layer of the medulla in each Example and Comparative Example (scale bar is 70 um). Arrows indicate sites where pathological findings are observed.

[Fig. 8]

Fig. 8 is a diagram showing typical pathological findings of basophilic renal tubules with inflammatory cell infiltration and fibrosis in each Example and Comparative Example (scale bar is 300 μm). Arrows indicate sites where pathological findings are observed.

[Fig. 9]

Fig. 9 is a diagram showing the results of SPECT-CT imaging using each $^{111}$In-labeled antibody. SPECT images of each $^{111}$In-labeled antibody are shown.

[Fig. 10]

Fig. 10 is a graph showing the results of VOI (volume of interest, three-dimensional ROI) analysis of the tumor (upper) and liver (lower) at each time point in SPECT images of each $^{111}$In-labeled antibody.

[Fig. 11]

Fig. 11 is a graph showing the results of confirmation of biodistribution (upper) and excretion pathway (lower) 168 hr after administration of respective $^{111}$In-labeled antibodies.

[Fig. 12]

Fig. 12 is an image showing the results of comparison of the binding ability of respective $^{111}$In-labeled antibodies by in vitro ARG.

[Fig. 13]

Fig. 13 shows graphs showing the results of the quantified and compared binding ability of respective $^{111}$In-labeled antibodies by in vitro ARG.

[Description of Embodiments]

Unless otherwise specified, the terms used in the present specification can be used with the meanings generally used in the pertinent technique field.

(1) $^{225}$Ac-labeled anti-MUC5AC humanized antibody

The $^{225}$Ac-labeled anti-MUC5AC humanized antibody used in the present invention uses Actinium-225 as a radioactive nuclide and is a conjugate of a chelating agent complexed with the radioactive nuclide and a humanized antibody that specifically binds to MUC5AC (hereinafter sometimes to be referred to as the conjugate of the present invention).

(1-1) Labeling with Actinium-225 ($^{225}$Ac labeling)

$^{225}$Ac, which is a radionuclide contained in the conjugate of the present invention, is known as a nuclide that emits α rays. $^{225}$Ac can be produced by a known method using an accelerator such as a cyclotron or a linear accelerator.

For example, it can be generated by a (p,2n) nuclear reaction by irradiating a $^{226}Ra$ target with protons using a cyclotron. The generated $^{225}Ac$ can be purified from the target by separation and purification. For example, purified $^{225}Ac$ can be obtained by dissolving a target containing $^{225}Ac$ with an acid or the like, adding an alkali to the dissolved solution to precipitate a salt containing $^{225}Ac$, and separating and purifying the salt. $^{225}Ac$ purified in this way can be used for RI labeling by chemically treating same as necessary to provide a chemical form suitable for RI labeling.

(1-2) Antibody

The antibody to be contained in the conjugate of the present invention is a humanized antibody that specifically binds to MUC5AC (hereinafter also to be referred to as the humanized antibody used in the present invention). The antibody may be used as an antigen-binding fragment thereof, and such embodiment is also encompassed in the present invention. Specifically, it contains a specific heavy chain variable region and a specific light chain variable region described below and, when desired, contains an appropriate heavy chain constant region and an appropriate light chain constant region. In the present specification, the "antigen-binding fragment" means an antibody fragment consisting of a part of the humanized antibody used in the present invention, and having the binding ability to MUC5AC. The number of amino acids contained in the polypeptide constituting the antigen-binding fragment is not particularly limited as long as it has the binding ability to MUC5AC.

Amino acid sequences preferred as the heavy chain variable region of the humanized antibody used in the present invention are shown below. The heavy chain variable region 1 (H01), heavy chain variable region 2 (H02), heavy chain variable region 3 (H03), and heavy chain variable region 4 (H04) respectively correspond to SEQ ID NOs: 1 to 4 in the Sequence Listing attached to the present specification. The underlined parts are CDR sites.

[heavy chain variable region (H01)]

E V Q L L E S G G G L V Q P G G S L R L S C A A S G F T F S N Y G M S W V R
Q A P G K G L E W V S T I S N S G R Y T Y F P D S V K G R F T I S R D N S K
N T L Y L Q M N S L R A E D T A L Y Y C T R H L D Y A N Y D A M D Y W G Q G
T L V T V S S

[heavy chain variable region (H02)]

L V Q L V E S G G G V V R P G G S L R L S C A A S G F T F S N Y G M S W I R
Q A P G K G L E W V S T I S N S G R Y T Y F P D S V K G R F T I S R D N A K
N S L Y L Q M N S L R A E D T A L Y Y C T R H L D Y A N Y D A M D Y W G Q G
T L V T V S S

[heavy chain variable region (H03)]

L V Q L V E S G G G V V Q P G R S L R L S C A A S G F T F S N Y G M S W V R
Q A P G K G L E W V A T I S N S G R Y T Y F P D S V K G R F T I S R D N S K
N T L Y L Q M N S L R A E D T A V Y Y C T R H L D Y A N Y D A M D Y W G Q G
T L V T V S S

[heavy chain variable region (H04)]

E V Q L L E S G G G L V Q P G G S L R L S C A V S G F T F S N Y G M S W V R
Q A P G K G L E W V S T I S N S G R Y T Y F P D S V K G R F T I S R D N S R
N T L Y L Q M N T L R A E D T A V Y Y C T R H L D Y A N Y D A M D Y W G Q G
T P V T V S S

Amino acid sequences preferred as the light chain variable region of the humanized antibody used in the present invention are shown below. The light chain variable region 1 (L01), light chain variable region 2 (L02), light chain variable region 3 (L03), and light chain variable region 4 (L04) respectively correspond to SEQ ID NOs: 5 to 8 in the Sequence

Listing attached to the present specification. The underlined parts are CDR sites.

[light chain variable region (L01)]

D I V M T Q S P S S L S A S V G D R V T I T C R A S K S V T T S D F S Y M H
W Y Q Q K P G K A P K L L I Y L A S N L E S G V P S R F S G S G S G T D F T
L T I S S L Q P E D F A T Y Y C Q H S R E F P W T F G G G T K V E I K

[light chain variable region (L02)]

D V V M T Q S P S T L S A S V G D R V T I T C R A S K S V T T S D F S Y M H
W Y Q Q K P G Q A P K L L I Y L A S N L E S G V P S R F S G S G S G T D F T
L T I S S L Q P E D F A T Y Y C Q H S R E F P W T F G Q G T K L E I K

[light chain variable region (L03)]

D I Q M T Q S P S S L S A S V G D R V T I T C R A S K S V T T S D F S Y M H
W Y Q Q K P G K S P K L L I Y L A S N L E S G V P S R F S G S G S G T D F S
L T I S S L Q P E D F A T Y Y C Q H S R E F P W T F G G G T K V E I K

[light chain variable region (L04)]

D I V M T Q S P D S L A V S L G E R A T I N C K A S K S V T T S D F S Y L H
W Y Q Q K P G Q P P K L L I Y L A S N L E S G V P D R F S G S G S G T D F T
L T I S S L Q A E D V A V Y Y C Q H S R E F P W T F G G G T K L E I K

In other words, the heavy chain variable region of the humanized antibody preferred in the present invention consists of the amino acid sequence shown in any one of SEQ ID NO: 1 to SEQ ID NO: 4, and the light chain variable region consists of the amino acid sequence shown in any one of SEQ ID NO: 5 to SEQ ID NO: 8. That is, the humanized antibody used in the present invention consists of a combination of any one of the above-mentioned four heavy chain variable regions (H01 - H04) and any one of the four light chain variable regions (L01 - L04).

A preferred humanized antibody in the present invention has heavy chain variable region H01, H03, or H04, and any one of L01 - L04 as the light chain variable region.

The most preferred humanized antibody in the present invention has heavy chain variable region H01 and light chain variable region L03.

The heavy chain variable region of the humanized antibody in the present invention is not limited to those defined by the amino acid sequence shown in SEQ ID NO: 1 to SEQ ID NO: 4 and also includes variants maintaining functions. That is, a mutated heavy chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 1 to SEQ ID NO: 4 is also used as the heavy chain variable region of the humanized antibody to be used in the present invention as long as it can bind to MUC5AC when combined with the light chain variable region in the present invention. An anti-MUC5AC humanized antibody having such amino acid sequence is a humanized antibody having the ability to specifically bind to MUC5AC, having stable property, and superior in tumor accumulation property.

In the present specification, the identity of the amino acid sequence refers to the identity of the amino acid sequences between the two proteins of interest, and is shown by the percentage (%) of amino acid residues that match in the optimal alignment of the amino acid sequences prepared using mathematical algorithms known in the pertinent technical field. The identity of an amino acid sequence can be determined by visual inspection and mathematical calculation, and can be calculated using a homology search program (e.g., BLAST, FASTA) or sequence alignment program (e.g., ClustalW) known to those skilled in the art, or genetic information processing software (e.g., GENETYX [registered trademark]), and the like. To be specific, the identity of the amino acid sequence in the present specification can be determined using systematic analysis program ClustalW (http://clustalw.ddbj.nig.ac.jp/index.php?lang=ja) published on the website of DDBJ (DNA DataBank of Japan) by the initial setting conditions (Version2.1, Alignment type: slow, DNA

Weight Matrix: Gonnet, GAP OPEN: 10, GAP EXTENSION: 0.1).

In addition, as the heavy chain variable region of the humanized antibody to be used in the present invention, a mutated heavy chain variable region consisting of the amino acid sequence shown in SEQ ID NO: 1 to SEQ ID NO: 4, wherein not more than 10, preferably not more than 8, further preferably not more than 5, most preferably not more than 3 (3, 2 or 1), amino acids are deleted, substituted, or added, is also used as the heavy chain variable region of the humanized antibody to be used in the present invention as long as it can bind to MUC5AC when combined with the light chain variable region in the present invention.

The light chain variable region of the humanized antibody to be used in the present invention is not limited to the amino acid sequence shown in SEQ ID NO: 5 to SEQ ID NO: 8 and also includes variants maintaining functions. That is, a mutated light chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 5 to SEQ ID NO: 8 is also used as the light chain variable region of the humanized antibody to be used in the present invention as long as it can bind to MUC5AC when combined with the heavy chain variable region in the present invention.

In addition, as the light chain variable region of the humanized antibody to be used in the present invention, a mutated light chain variable region consisting of the amino acid sequence shown in SEQ ID NO: 5 to SEQ ID NO: 8, wherein not more than 10, preferably not more than 8, further preferably not more than 5, most preferably not more than 3 (3, 2 or 1), amino acids are deleted, substituted, or added, is also encompassed in the light chain variable region of the humanized antibody to be used in the present invention as long as it can bind to MUC5AC when combined with the heavy chain variable region in the present invention.

The humanized antibody to be used in the present invention can be produced by a method generally performed in the art or a method analogous thereto. Specifically, the following steps can be performed.

Since the amino acid sequences of the heavy chain variable region and the light chain variable region of the humanized antibody to be used in the present invention are disclosed in SEQ ID NO: 1 to SEQ ID NO: 8, a nucleic acid encoding the antibody obtained based on the amino acid sequence information is constructed and inserted into a suitable expression vector. The expression vector can optionally contain, in addition to the nucleic acid encoding the humanized antibody to be used in the present invention, Kozak sequence to improve translation efficiency, a signal sequence that promotes secretion of the humanized antibody to be used in the present invention into the medium when introduced into a host, a promoter sequence, and the like. The vector that can be used in the present invention can be selected from those generally used in the pertinent technical field, and plasmid vector pcDNA3.4 is preferred. Introduction of an expression vector into the host cell is not particularly limited. As a method for introducing a gene into a cell, a method conventionally used in the pertinent technical field, for example, a method known to those skilled in the art such as calcium phosphate method, electroporation method, lipofection method, and DEAE-dextran method can be used. An introduction method using the lipofection method is particularly preferred, as performed in the following Example. As the host cell used for this purpose, those conventionally used in the pertinent technical field can be used. Examples of such host cell include CHO cell, 293 cell, Escherichia coli, Pichia yeast, Sf9 cell and the like. Currently, an expression system kit for expressing the protein of interest is also commercially available. The ExpiCHO System (manufactured by Thermo Fisher Scientific) used in the following Example is particularly preferred for rapid and reliable expression of the protein of interest.

The humanized antibody to be used in the present invention can be obtained by inserting a nucleic acid encoding the humanized antibody to be used in the present invention into an expression vector, introducing the nucleic acid into a host cell by the expression vector containing the nucleic acid, culturing the host cell after introduction of the nucleic acid, and obtaining the humanized antibody of the present invention from the culture supernatant thereof by a purification means such as chromatography and the like. In this method, the humanized antibody to be used in the present invention is secreted in a culture supernatant by culturing the host cell. The humanized antibody or an antigen-binding fragment thereof to be used in the present invention can be obtained from the culture supernatant by using a purification means such as chromatography, and the like. As the means for chromatography, various means known in the pertinent technical field such as affinity chromatography, ion exchange chromatography, size-exclusion chromatography and the like can be used. Affinity chromatography with the protein A column used in the following Example is particularly preferred.

The above-mentioned humanized antibody may be a polyclonal antibody or a monoclonal antibody.

(1-3) Chelating agent

In the present invention, the chelating agent is not particularly limited as long as it has a site in the structure thereof where Actinium-225 is coordinated. Preferably, it has a chelate site, which is a site to which Actinium-225 is coordinated, and a substituent that enables conjugating with an antibody. Examples of the chelate site include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α'',α'''-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-Carboxyethyl)-1,4,7,10-tetraazacyclododecane-1,4,7,10-tetraacetic acid), DOTA-GA-NHS, DOTP (((1,4,7,10-Tetraazacyclododecane-1,4,7,10-tetrayl)tetrakis(methylene))tetraphosphonic acid), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane ($L^{py}$), p-SCN-Bn-DOTA (S-2-(4-Isothiocyanatobenzyl)-1,4,7,10-tetraazacyclododecane tetraacetic acid), MeO-DOTA-NCS (1-[(2-methoxy-5-isothiocyanato phenyl)-carboxymethyl]-4,7,10-triscarboxy 5 methyl-1,4,7,10-tetraazacyclododecane), EuK-106, DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), DTPA-BMA (5,8-Bis(carboxymethyl)-11-[2-(methylamino)-2-oxoethyl]-3-oxo-2,5,8,11-tetraazatridecan-13-oic acid), EDTA (2,2',2'',2'''-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid), TETPA (1,4,8,11-tetraazacyclotetradecane-1,4,8,11-tetrapropionic acid), TETA (1,4,8,11-Tetraazacyclotetradecane-N,N',N'',N'''-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N'',N'''-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N'',N''',N''''-penta-acetic acid), H4octapa (N,N'-bis(6-carboxy-2-pyridylmethyl)-ethylenediamine-N,N'-diacetic acid), H2bispa2 (6,6'-({9-hydroxy-1,5-bis (methoxycarbonyl)-2,4-di (pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N''-bis(6-carboxy-2-pyridylmethyl)-diethylenetriamine-N,N',N''-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), porphyrin, DO3A (1,4,7,10-Tetraazacyclododecane-1,4,7-triacetic acid trisodium salt), and DO3A-NHS (1,4,7,10-Tetraazacyclododecane-1,4,7,10-tetraacetic acid mono-N-hydroxysuccinimide ester).. It is preferable to have a structure derived from a compound represented by the following formula (A).

(A)

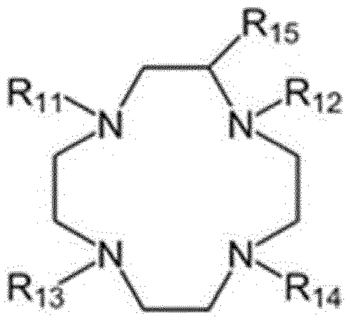

In the formula (A), $R_{11}$, $R_{13}$ and $R_{14}$ are each independently a group consisting of - $(CH_2)_pCOOH$, -$(CH_2)_pC_5H_5N$, - $(CH_2)_pPO_3H_2$, - $(CH_2)_pCONH_2$ or - (CHCOOH) $(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the aforementioned antibody, p is an integer of not less than 0 and not more than 3, $R_{15}$ is a hydrogen atom when $R_{12}$ is a substituent for conjugating with the aforementioned antibody, and $R_{15}$ is a substituent for conjugating with the aforementioned antibody when $R_{12}$ is not a substituent for conjugating with the aforementioned antibody.

Examples of the specific structure represented by the formula (A) include structures derived from the compounds represented by the following formulas (A-1) to (A-12).

(A-1)

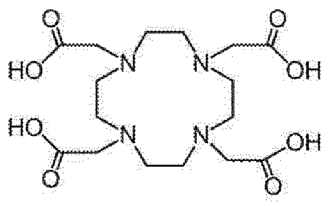

DOTA

(A-2)

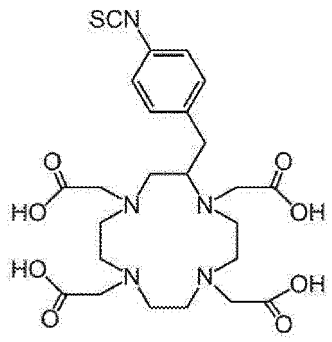

p-SCN-Bn-DOTA

(A-3)

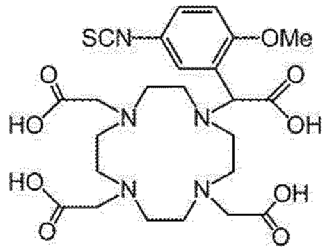

MeO-DOTA-NCS

(A-4)

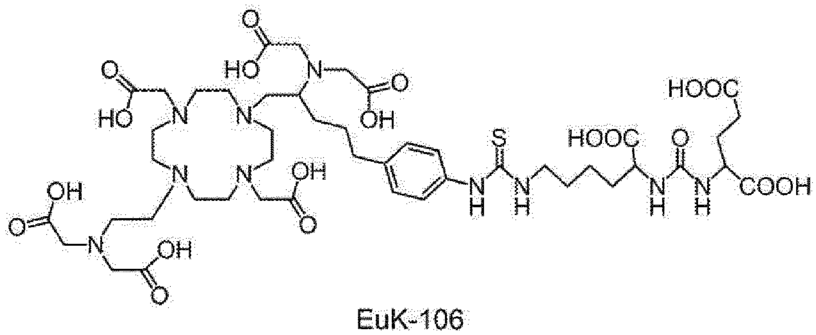

EuK-106

(A-5)

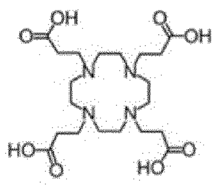

DOTPA

(A-6)

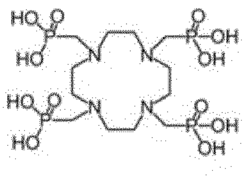

DOTMP

(A-7)

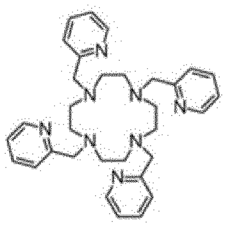

$L^{py}$

(A-8)

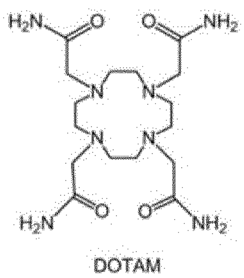

DOTAM

(A-9)

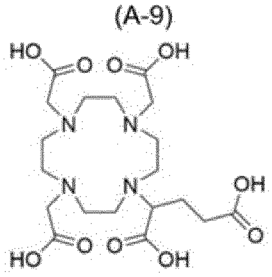

DOTA-GA

(A-10)

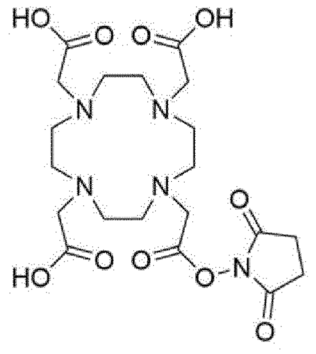

DO3A-NHS

(A-11)

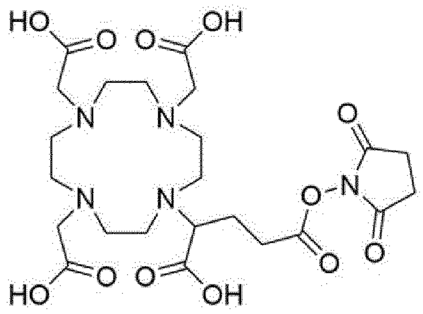

DOTA-GA-NHS

(A-12)

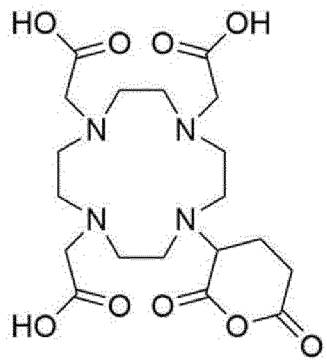

DOTA-GA-anhydride

The linkage site between the chelate site and the substituent that enables formation of a conjugate with the antibody is preferably an amide bond or a thiourea bond, more preferably an amide bond from the aspect of stability.

The amide bond can be formed, for example, by the reaction of an N-hydroxysuccinimidoester (NHS) group of the above-mentioned formula (A-10) or (A-11), or a 2,6-dioxo tetrahydro-2H-pyranyl group of the above-mentioned (A-12) with primary amine. The thiourea bond can be formed by the reaction of an isothiocyanate group of the compound of the above-mentioned formula (A-2), (A-3) with primary amine or a maleimide group.

In the conjugate of the present invention, the chelating agent may be provided at least not less than one molecule, preferably not less than 1 molecule and not more than 8 molecules, per one molecule of the antibody. To maintain the activity of the antibody itself (antigen recognition action, neutralizing action, complement activating action and/or opsonin action), a chelating agent is preferably introduced site-specifically into the Fc region (constant region) of the antibody. In the present invention, the chelating agent is preferably provided at one or two molecules per one molecule of the antibody.

In the conjugate of the present invention, the chelating agent may be connected to the antibody via a linker. Examples of the linker include substituted or unsubstituted alkyl group, substituted or unsubstituted heteroalkyl group, polyethylene glycol (PEG) group, peptides, sugar chain, disulfide group, combination of these and the like.

Preferably, the chelating agent modifies the antibody site-specifically, more preferably in the Fc region, via a linker. In this case, the linker contains a peptide consisting of not less than 13 and not more than 17 amino acid residues represented by the following formula (i) (hereinafter, to be also referred to as "antibody-modification peptide"), and one formed by a cross-linking reaction between the antibody-modification peptide modified with a crosslinking agent and the antibody can be used. In the explanation of the formula (i), the left side of the paper surface of the amino acid sequence indicates the N-terminal side, and the right side of the paper surface of the amino acid sequence indicates the C-terminal side. When the chelating agent is connected to the antibody via the antibody-modification peptide as a linker, the position where the chelating agent and the antibody-modification peptide are linked is not particularly limited. For example, it can be directly or indirectly linked at the N-terminal or C-terminal of the antibody-modification peptide, preferably at the N-terminal. In addition, the C-terminal of the antibody-modification peptide may be modified by, for example, amidation or the like to improve its stability and the like.

$$(Xa)\text{-}Xaa1\text{-}(Xb)\text{-}Xaa2\text{-}(Xc)\text{-}Xaa3\text{-}(Xd)\cdots \qquad (i)$$

In the formula (i), Xa, Xb, Xc and Xd are each continuous X in the number of a, continuous X in the number of b, continuous X in the number of c, and continuous X in the number of d, respectively,

X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa1 and Xaa3 are linked, and
Xaa2 is a lysine residue, arginine residue, cysteine residue, aspartic acid residue, glutamic acid residue, 2-aminosuberic acid, or diamino propionic acid, and modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

Xaa1 and Xaa3 are amino acid residues derived from an amino acid having a thiol group in the side chain, and Xaa1 and Xaa3 may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the wavy line indicates the binding part with the sulfide group.

(4) 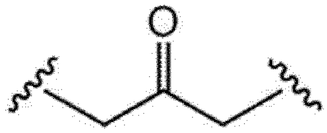

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Among these, the amino acid sequence of the antibody-modification peptide preferably has any one of the following sequences (1) to (14), more preferably the following sequence (1), (2), (13) or (14). In the following amino acid sequences (1) to (14), (Xaa2) is a lysine residue, a cysteine residue, an aspartic acid residue, a glutamic acid residue, a 2-aminosuberic acid, or a diamino propionic acid, and (Xaa1) and (Xaa3) are each a homocysteine residue. In the following amino acid sequences (1) to (14), the amino acids other than (Xaa1), (Xaa2) and (Xaa3) are indicated by one-letter abbreviations.

(1) DCAYH(Xaa2)GELVWCT (SEQ ID NO: 9)
(2) GPDCAYH(Xaa2)GELVWCTFH (SEQ ID NO: 10)
(3) RCAYH(Xaa2)GELVWCS (SEQ ID NO: 11)
(4) GPRCAYH(Xaa2)GELVWCSFH (SEQ ID NO: 12)
(5) SPDCAYH(Xaa2)GELVWCTFH (SEQ ID NO: 13)
(6) GDDCAYH(Xaa2)GELVWCTFH (SEQ ID NO: 14)
(7) GPSCAYH(Xaa2)GELVWCTFH (SEQ ID NO: 15)
(8) GPDCAYH(Xaa2)GELVWCSFH (SEQ ID NO: 16)
(9) GPDCAYH(Xaa2)GELVWCTHH (SEQ ID NO: 17)
(10) GPDCAYH(Xaa2)GELVWCTFY (SEQ ID NO: 18)
(11) SPDCAYH(Xaa2)GELVWCTFY (SEQ ID NO: 19)
(12) SDDCAYH(Xaa2)GELVWCTFY (SEQ ID NO: 20)
(13) RGNCAYH(Xaa2)GQLVWCTYH (SEQ ID NO: 21)
(14) G(Xaa1)DCAYH(Xaa2)GELVWCT(Xaa3)H (SEQ ID NO: 22)

(1-4) Production method of conjugate

The production method of the conjugate of the present invention includes two steps which are a conjugation step of conjugating a chelating agent and an antibody, and a complex formation step of forming a complex of Actinium-225 and a chelating agent. The conjugation step may be performed before the complex formation step or after the complex formation step.

In the conjugation step, various methods for chemical modification of antibody are used. Specifically, the methods (a) - (f) can be mentioned:

(a) amine coupling method (a method for modifying the amino group of a lysine residue of an antibody by using a chelating agent or chelate having a carboxyl group activated by an N-hydroxysuccimidyl (NHS) group)
(b) method for modifying a sulfhydryl (SH) group generated by partially reducing a disulfide bond (SS bond) between polypeptide chains at the hinge site of an antibody with a chelating agent or linker having a maleimide group reactive with the SH group
(c) method for modifying cysteine newly introduced into an antibody by an amino acid mutation by genetic engineering with a chelating agent or linker having a maleimide group
(d) method for modifying an azide group of azidized lysine newly introduced into an antibody by an amino acid mutation by genetic engineering with a chelating agent or linker having alkyne (e.g., Dibenzylcyclooctyne: DBCO) by using a click reaction
(e) method for modifying glutamine introduced into a specific position of an antibody with a chelating agent or linker having a side chain of lysine by using transglutaminase
(f) method of site-specifically modifying the Fc region of an antibody with a chelating agent or linker having the antibody-modification peptide shown in the aforementioned (i).

In the complex formation step, the chelating agent is chelated with Actinium-225 (complex formation). The Actinium-225 used here is preferably used in a manner permitting ionization, more preferably in the form of an ion, from the viewpoint of increasing the complex formation efficiency. In the complex forming step, the order of addition of the radionuclide to the chelating agent does not matter as long as a complex can be formed with the radionuclide. For example, a solution in which radioactive metal ion is dissolved in a solvent mainly composed of water can be used as a radionuclide.

After complex formation, the obtained complex may be purified using a filtration filter, a membrane filter, a column filled with various fillers, chromatography or the like.

In the production method of the conjugate of the present invention, a conjugation step is preferably performed after the complex formation step.

In a more preferred embodiment, in complex formation step (A), a complex is formed between Actinium-225 and a chelating agent having a first atomic group capable of click reaction as a substituent for enabling conjugation with the antibody. Then, in conjugation step (B), using an antibody-modification peptide shown by the aforementioned (i) and an antibody-modification linker having a second atomic group capable of click reaction, a click reaction is performed between the peptide-modified antibody in which Fc region is site-specifically modified and the chelating agent with a formed complex which is obtained in step (A) to obtain the conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker is preferably alkyne and the antibody-modification linker is preferably azide, or the chelate linker is preferably 1,2,4,5-tetrazine and the antibody-modification linker is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), and a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a).

(1a) 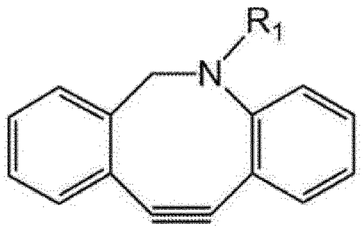

(2a) 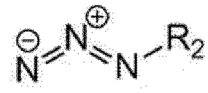

Dibenzylcyclooctyne

Azide

wherein $R_1$ is a linkage site with a chelating agent, and $R_2$ is a linkage site with an antibody-modification peptide in the antibody.

(1b) 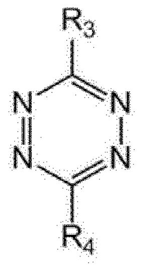

1,2,4,5-tetrazine

(2b) 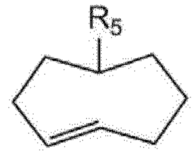

*trans*-cyclooctene

wherein one of $R_3$ and $R_4$ is a linkage site with a chelating agent or an antibody-modification peptide in the antibody, and the other is a hydrogen atom, a methyl group, a phenyl group or a pyridyl group, and $R_5$ is a linkage site with any one chelating agent or an antibody-modification peptide in the antibody depending on $R_3$ or $R_4$.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In step (A), more preferably, a chelating agent having a structure represented by the following formula (ii) is used.

A-B-C ··· (ii)

In the formula (ii), A is a chelate site represented by the following formula (iia).

(iia) 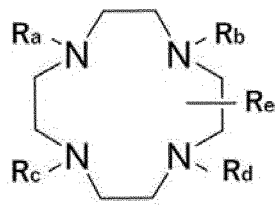

In the formula (iia), Ra, Rb, and Rc are each independently a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, p is an integer of not less than 0 and not more than 3, either Rd or Re is a binding site (*) to B, and the other is a hydrogen atom or a group consisting of $-(CH_2)_pCOOH$, $-(CH_2)_pC_5H_5N$, $-(CH_2)pPO_3H_2$, $-(CH_2)_pCONH_2$, or $-(CHCOOH)(CH_2)_pCOOH$, and p is an integer of not less than 0 and not more than 3.

In the formula (ii), B is represented by the following formula (iib):

(iib) 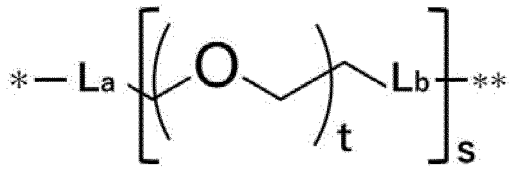

In the formula (iib), La and Lb are each independently a bond linker containing at least an amide bond or thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

(iic) 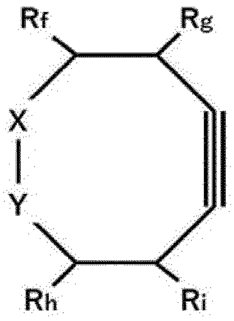

(iid) 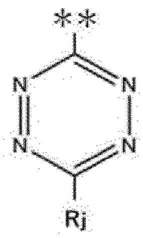

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (iia) wherein Ra to Rd are $-(CH_2)_pCOOH$, p is 1, Re is a binding site with B; or DO3A derivative or DOTAGA derivative wherein Ra to Rc are - $(CH_2)_pCOOH$, p is 1, Rd is a binding site with B, and Re is a hydrogen atom is more preferred.

In the formula (ii), a DOTA-PEGt-DBCO derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is an alkyne derivative represented by the formula (iic), wherein, in the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B; or a DOTA-PEGt-Tz derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is a tetrazine derivative represented by the formula (iid) is further preferred.

In the formula (ii), a DO3A-PEGt-DBCO derivative wherein A is the above-mentioned DO3A derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the formula (ii), a DOTAGA-PEGt-DBCO derivative wherein A is the above-mentioned DOTAGA derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the molar ratio of the chelating agent and Actinium-225 as chelate site/Actinium-225, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer, and the like, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

In the reaction time, provided that the reaction temperature is as described above, lower limit is preferably not less than 5 minutes, more preferably not less than 10 minutes, further preferably not less than 20 minutes, further more preferably not less than 30 minutes, particularly preferably not less than 45 minutes, and the upper limit is preferably not more than 180 minutes, more preferably not more than 150 minutes, further preferably not more than 120 minutes, further more preferably not more than 90 minutes, and especially not more than 60 minutes, for example, and a range of not less than 10 minutes and not more than 150 minutes is preferred, not less than 10 minutes and not more than 60 minutes is more preferred.

The antibody to be used in step (B) is a peptide-modified antibody in which Fc region (constant region) of humanized antibody as described in detail in the above-mentioned "(1-2) Antibody" is site-specifically modified using the antibody-modification peptide shown in the aforementioned (i), and an antibody-modification linker having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. The se methods can be performed according to the method described in, for example, WO 2017/217347 and WO 2018/230257.

The antibody-modification linker may be one in which an antibody-modification peptide and a linker represented by the following formula (S1) are bonded.

$$*\text{-}((L_1)_m\text{-}Z)_k\text{-}L_2\text{-}AG_2 \qquad (S1)$$

wherein * is a binding site with the N-terminal or C-terminal of peptide,

$L_1$ is a linker moiety of polyethylene glycol (PEG),
m is an integer of not less than 1 and not more than 50,
Z is a second linker moiety that binds $(L_1)_m$ and $L_2$,
k is 0 or 1,
$L_2$ is the second PEG linker moiety, and
$AG_2$ is a second atomic group.

In the aforementioned formula (S1), the structure of Z is not particularly limited as long as it is a linker structure that binds $(L_1)_m$ and $L_2$ to each other, and includes, for example, an amino acid sequence consisting of not less than 1 and not more than 5 amino acid residues. In this case, the amino acid sequence contained in Z preferably contains a cysteine residue, and is more preferably bonded to $L_2$ via a thioether group formed by the bond between the thiol group of the cysteine residue and a maleimide group.

In the present invention, the PEG linker moiety constituting $L_2$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

(P2) 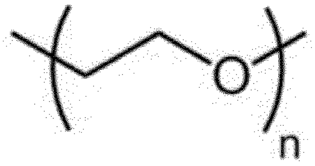

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker, an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an antibody to obtain a peptide-modified antibody can be performed, for example, using a crosslinking agent. A crosslinking agent is a chemical substance for linking an antibody-modification peptide and an antibody by a covalent bond. Examples thereof include a crosslinking agent preferably containing two or more succinimidyl groups such as disuccinimidyl glutarate (DSG), disuccinimidyl suberate (DSS) and the like, a crosslinking agent consisting of a compound containing two or more imidic acid moieties such as dimethyl adipimidate and the like, or a salt thereof, a crosslinking agent consisting of a compound having a disulfide bond such as dimethyl 3,3'-dithiobispropionimidate, dithiobissuccinimidylpropionic acid, and the like, or a salt thereof, and the like. Using such crosslinking agent, a crosslinking reaction can be caused between an amino acid residue of Xaa2 in the antibody-modification peptide and an antibody. When, for example, the humanized antibody of the present invention is used as the antibody, the crosslinking reaction in the antibody occurs site-specifically between an amino acid residue of Xaa2 and a Lys252 residue according to the Eu numbering in the humanized antibody of the present invention. These Lys residues are present in the Fc region of the humanized antibody of the present invention.

The method for binding the antibody-modification peptide to the antibody can be performed, for example, by dispersing the aforementioned antibody-modification peptide, an antibody, a crosslinking agent, and a catalyst as necessary in an appropriate buffer at not less than 10°C and not more than 30°C. The reaction time may be about not less than 10 min to 2 hr. The molar ratio at the time of reaction of the peptide and the antibody, as the antibody/peptide, is preferably not less than 1/5, more preferably not less than 1/3, further preferably not less than 1/1.5 as the lower limit of the antibody/peptide, and the upper limit is preferably not more than 20/1, more preferably not more than 10/1, further preferably not more than 5/1, further more preferably not more than 1/1, particularly preferably not more than 1/1.7. For example, the range of not less than 1/5 and not more than 20/1 is preferable, and not less than 1/1.5 and not more than 1/1.7 is more preferable.

The peptide-modified antibody obtained through the above steps is a mixture containing an antibody in which

one molecule of antibody-modification peptide is bound to one molecule of an antibody (hereinafter to be referred to as "monovalent antibody") and an antibody in which two molecules of antibody-modification peptide are bound to one molecule of an antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is preferable to use a column packed with various fillers, and it is more preferable to use a column packed with a filler suitable for separation and purification of proteins such as antibody and the like.

The filler suitable for separation and purification of protein such as antibody and the like is not particularly limited as long as it is a filler in which an immunoglobulin-binding protein is immobilized on a carrier composed of a water-insoluble substrate, and which specifically binds to an antibody. Examples of the immunoglobulin-binding protein include protein A, protein G, protein L and the like. These immunoglobulin-binding proteins may be genetically engineered recombinants. Examples of the recombinant immunoglobulin-binding protein include genetically-engineered protein A, genetically-engineered protein G, and fused protein A domain and protein G domain. In the present invention, as a filler suitable for separation and purification of at least a monovalent antibody and a divalent antibody, protein A is more preferred, and genetically-engineered protein A is more preferred. As used herein, protein A and protein G are protein molecules that can specifically bind to an antibody molecule IgG, and classified as protein A (Staphylococcus aureus) or protein G (streptococcus: Streptococcus genus) depending on the difference in the origin of the isolated microorganisms. The genetically-engineered protein A is a protein A in which at least one amino acid mutation has been introduced into an amino acid residue of any of the IgG binding domains (E, D, A, B and C domains) of protein A. In the present invention, genetically-engineered protein A in which the domain into which at least one amino acid mutation has been introduced is multimerized is preferable, genetically-engineered protein A in which A, B or C domain into which at least one amino acid mutation of protein A has been introduced is multimerized is more preferable, and genetically-engineered protein A multimerized to not less than a dimer and not more than a pentamer is further more preferable. The amino acid mutation may be derived from any mutation such as substitution, deletion, insertion and the like of the amino acid sequence or the base sequence encoding the amino acid in the transcriptional translation step of the gene. Examples thereof that are not particularly limited include the genetically-engineered protein A described in WO 2003/080655, WO 2011/118699 and the like.

Examples of the water-insoluble substrate on which immunoglobulin-binding proteins are immobilized include inorganic carriers such as glass beads, silica gel and the like, organic carriers such as synthetic polymers (e.g., crosslinked poly(vinyl alcohol), crosslinked polyacrylate, crosslinked polyacrylamide, crosslinked polystyrene) and polysaccharides (e.g., crystalline cellulose, crosslinked cellulose, crosslinked agarose, crosslinked dextran), and organic-organic, organic-inorganic conjugate carriers and the like obtained from combinations of these, and the like.

The column filled with the aforementioned genetically-engineered protein A as a filler is commercially available as, for example, KanCap (registered trademark) series (KANEKA KanCapA prepacked column) of KANEKA CORPORATION, HiTrap (registered trademark) series (HiTrap Mabselect, HiTrap Mabselect SuRe, HiTrap Mabselect Xtra) of GE Healthcare, HiScreen series (HiScreen Mabselect SuRe) of GE Healthcare, TOYOPEARL (registered trademark) series (TOYOPEARL AF-rProtein A-650F) of Tosoh Corporation, and the like.

The separation and purification of peptide-modified antibody used for click reaction in step (B) is explained below as an example.

A peptide-modified antibody is subjected to a click reaction in step (B) after an antibody modification step in which a modified antibody is obtained by site-specifically modifying an Fc region of an antibody by a linker provided with an antibody-modification peptide (antibody-modification linker), and an antibody purification step in which the modified antibody is purified using the aforementioned carrier with an immunoglobulin-binding protein immobilized thereon. In addition, the antibody purification step further includes a retention step of retaining the modified antibody retained on the carrier, a washing step of washing the modified antibody not retained on the carrier, and an elution step of eluting the modified antibody retained on the carrier in the retention step.

More specifically, in the antibody modification step, a modified antibody is obtained as a mixture containing an unmodified antibody not modified by an antibody-modification linker, a monovalent antibody, and a divalent antibody and, in the antibody purification step, the first antibody composition containing relatively large amounts of the unmodified antibody and the monovalent antibody and the second antibody composition containing a relatively large amount of the divalent antibody are respectively eluted utilizing the difference in the interaction of the unmodified antibody, monovalent antibody and divalent antibody with immunoglobulin-binding proteins. That is, in the retention step and washing step among the antibody purification steps, the second antibody composition containing a relatively large amount of peptide-modified antibody (divalent antibody) having a low degree of interaction with immunoglobulin-binding proteins is eluted and, in the elution step among the antibody purification steps, the first antibody composition containing a relatively large amount of peptide-modified antibody (unmodified antibody and monovalent antibody) having a high degree of interaction with immunoglobulin-binding proteins is eluted. As used herein, "containing a relatively large amount of unmodified antibody and monovalent antibody" means that the total amount of unmodified antibody and monovalent antibody contained in the first antibody composition is larger than that of the divalent antibody contained in the antibody composition, preferably that the total amount of the unmodified antibody and monovalent antibody is not less than 55%, not less than 63%, not less than 70%, not less than 80%, or not less than 90%, of the total amount (100%) of the unmodified antibody and modified antibody contained in the antibody composition. In addition, "containing a relatively large amount of divalent antibody" means that the amount of divalent antibody contained in the second antibody composition is larger than that of the monovalent antibody contained in the antibody composition, preferably that the amount of divalent antibody is not less than 55%, not less than 63%, not less than 70%, not less than 80%, or not less than 90%, of the total amount (100%) of the unmodified antibody and modified antibody contained in the antibody composition.

In the retention step, a solution containing the mixture of the unmodified antibody, monovalent antibody and divalent antibody obtained in the antibody modification step is added to a column, and unmodified antibody and monovalent antibody retained on the carrier are retained on the column and the divalent antibody not retained on the carrier is allowed to pass through. The solution that passed through in the retention step constitutes a part of the second antibody composition. To facilitate retention of the unmodified antibody and monovalent antibody on the column and to prevent aggregation or denaturation of these, it is preferable to dilute the mixed solution of the peptide-modified antibody with an appropriate dilution solvent and add same to the column. The dilution solvent is not particularly limited as long as the peptide-modified antibody dissolves and does not easily aggregate or denature in the solvent, and water, saline, buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. When a buffer is used as a dilution solvent, the concentration of the buffering agent is not less than 10 mmol/L, preferably not less than 15 mmol/L, more preferably not less than 20 mmol/L as the lower limit, and not more than 1000 mmol/L, preferably not more than 500 mmol/L, more preferably not more than 100 mmol/L as the upper limit. In addition, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the washing step, the modified antibody remaining in the column is eluted from the column by using a wash solvent. The solution that passed through the column in the aforementioned retention step and the solution eluted from the column in the washing step contain a relatively large amount of the divalent antibody, and therefore, these can be combined and used as the second antibody composition.

The wash solvent is not particularly limited as long as the peptide-modified antibody dissolves and does not easily aggregate or denature in the solvent, and it is a buffer having appropriate pH buffering capacity, and buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. The concentration of the buffering agent used as the wash solvent is not less than 20 mmol/L, preferably not less than 30 mmol/L as the lower limit, and not more than 200 mmol/L, preferably not more than 70 mmol/L as the upper limit. The pH of the wash solvent is not less than 4.0, preferably not less than 4.5, more preferably not less than 4.8 as the lower limit, and not more than 7.4, preferably not more than 6.0, more preferably not more than 5.2 as the upper limit. Furthermore, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the elution step, the modified antibody retained on the carrier is eluted from the column by using an elution solvent. That is, the first antibody composition containing a relatively large amount of unmodified antibody and monovalent antibody is eluted from the column by using an elution solvent.

As the elution solvent, a buffer such as sodium acetate buffer, ammonium acetate buffer, citrate buffer and the like can be used. In addition, to reduce non-specific binding to the antibody-modification linker, unmodified antibody and modified antibody column carrier, the elution solvent may contain an additive such as sodium chloride, potassium chloride and the like. The concentration of the additive contained in the elution solvent is not particularly limited and may be, for example, 0.15 mol/L.

When the elution solvent contains a buffering agent, the concentration of the buffering agent is not less than 20 mmol/L, preferably not less than 30 mmol/L as the lower limit, and not more than 200 mmol/L, preferably not more than 70 mmol/L as the upper limit. In addition, to weaken the interaction between the unmodified antibody and monovalent

antibody, and the immunoglobulin-binding protein, and to prevent denaturation and aggregation of the antibody, the pH of the elution solvent is not less than pH 3.0 as the lower limit, and not more than pH 4.2 as the upper limit.

The first antibody composition or the second antibody composition obtained in the antibody purification step may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the conjugate obtained in step (A) are capable of click reaction, the order of addition of these does not matter. For example, one of the conjugate and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the conjugate and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferred, and the range of not less than 0.1 mL and not more than 10 mL is more preferred.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferred, and the range of not less than 1 μmol/L and not more than 100 μmol/L is more preferred, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferred, and the range of not less than 10 min and not more than 20 hr is more preferred.

The obtained conjugate may be used as it is or purified using a filtration filter, a membrane filter, a column filled with various fillers, chromatography or the like.

In the conjugate produced by steps (A) and (B), a specific site of a humanized antibody that specifically binds to MUC5AC (e.g., the lysine residue in the Fc region of antibody) is specifically modified with a chelating agent. This conjugate comprises one or two molecules of the aforementioned chelating agent per one molecule of the antibody. The chelating agent site-specifically modifies the Fc region of the antibody of the present invention via a linker. The linker is constituted of a chelate linker that connects to a chelating agent, a first atomic group that connects to the linker, a second atomic group that can perform click reaction with the first atomic group, and an antibody-modification linker that connects to the second atomic group (including an antibody-modification peptide represented by the above-mentioned formula (i)). Therefore, the linker has a chemical structure derived from the first atomic group and the second atomic group. As such chemical structure, a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

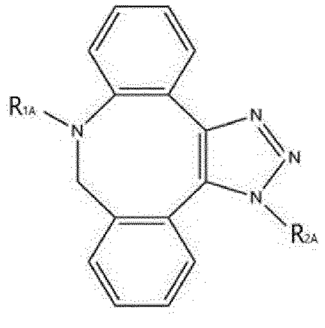

(10a)

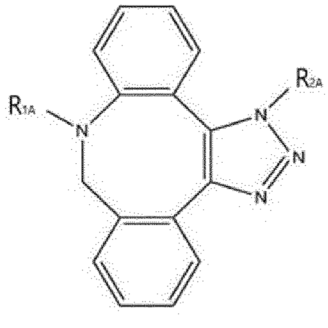

(10b)

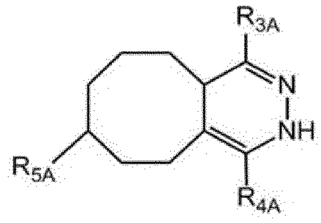

(10c)

In the formula (10a) and the formula (10b), $R_{1A}$ is a binding site with a chelate linker, and $R_{2A}$ is a binding site with an antibody-modification linker. In the formula (10c), one of $R_{3A}$ and $R_{4A}$ is a hydrogen atom, a methyl group, a phenyl group or a pyridyl group, and the other is a binding site with a chelate linker, and $R_{5A}$ is a binding site with an antibody-modification linker.

(1-5) Drug for reducing renal toxicity

The present invention is characterized in that the $^{225}$Ac-labeled anti-MUC5AC humanized antibody is used in combination with a drug for reducing renal toxicity (hereinafter to be also referred to as a renal protective agent).

Among the daughter nuclides of $^{225}$Ac, $^{221}$Fr and $^{217}$At have short half-lives (4.9 min and 32.3 msec, respectively), and they are considered to decay at the same distribution sites as $^{225}$Ac. On the other hand, since $^{213}$Bi has a long half-life (46 min) compared to other daughter nuclides, it has been reported that it may be redistributed within the body after decay and accumulate in the kidney, which is the physiological accumulation organ of bismuth. Therefore, when administering $^{225}$Ac-labeled antibody, as one measure to prevent renal toxicity caused by the administration of a drug containing Actinium-225 or from Actinium-225 or daughter nuclides of Actinium-225, it is considered important to reduce the accumulation in the kidney of $^{213}$Bi produced in vivo. Thus, the renal protective agent used in the present invention is not particularly limited as long as it is a drug capable of reducing the accumulation of radionuclides and/or daughter nuclides thereof, preferably $^{213}$Bi, in the kidney. For example, antihypertensive agents, anticoagulants (heparin, warfarin, etc.), antiplatelet agents (dipyridamole, diraceb hydrochloride, etc.), oral adsorbents (spherical adsorbed charcoal, etc.), steroids (dexamethasone, prednisolone, etc.), immunosuppressants (mizoribine, cyclosporine, etc.), and the like can be mentioned. Examples of the antihypertensive agent include angiotensin II receptor antagonists (e.g. losartan, candesartan, irbesartan, valsartan, eprosartan, telmisartan), angiotensin converting enzyme inhibitors (e.g. enalapril, lisinopril, benazepril, imidapril, captopril, enalaprilat, alacepril, perindopril erbumine, delapril, quinapril, ramipril, cilazapril, fosinopril, trandolapril, temocapril, moexipril, spirapril, zofenopril), Ca antagonists (e.g., amlodipine, nifedipine, nicazipine, diltiazem, manidipine, benidipine, nilvadipine, nitrendipine, nisoldipine, balnidipine), metal scavengers (e.g. DTPA, Ca-DTPA or Zn-DTPA), diuretics (e.g. spironolactone, hydrochlorothiazide, furosemide, triamterene, eplerenone), $\alpha$ blockers (e.g. doxazosin, prazosin, bunazosin, terazosin, urapidil), $\beta$-blockers (e.g., metoprolol, atenolol, bisoprolol, arotinolol, celiprolol, betaxol), and the like.

The renal protective agent may be a pharmaceutically acceptable prodrug of the above-mentioned compound, or a pharmaceutically acceptable salt thereof.

It is preferably a diuretic or a metal scavenger, more preferably a metal scavenger selected from DTPA, Ca-DTPA, and Zn-DTPA, or a diuretic selected from furosemide, spironolactone, and eplerenone. It is more preferably Zn-DTPA or furosemide.

(1-6) Radiopharmaceutical (radioactive antitumor agent)

The conjugate produced by the method shown in the aforementioned (1-4) ($^{225}$Ac-labeled anti-MUC5AC humanized antibody) can be used as a radioactive antitumor agent (hereinafter to be also referred to as radioactive antitumor agent 1 in the present specification) in combination with the drug for reducing renal toxicity (renal protective agent) described in the aforementioned (1-5). As another embodiment of the present invention, a radioactive antitumor agent (hereinafter to be also referred to as radioactive antitumor agent 2 in the present specification) containing the conjugate produced by the method shown in the aforementioned (1-4) ($^{225}$Ac-labeled anti-MUC5AC humanized antibody) and the drug for reducing renal toxicity (renal protective agent) described in the aforementioned (1-5) as active ingredients can be used.

The amount of the $^{225}$Ac-labeled anti-MUC5AC humanized antibody in the radioactive antitumor agent 1 or the

radioactive antitumor agent 2 of the present invention varies depending on the administration form of the radioactive antitumor agent (described later), the severity of the disease, the animal species to be the subject of administration, and drug acceptability, weight, age, and the like of the subject of administration. Generally, for adults, it can be administered at a dose of not more than 250 kBq/kg per dose. It can be effective even at a dose of not more than 80 kBq/kg per dose. In this case, generally, the dose of the renal protective agent used in combination is not particularly limited as long as it is a dose used clinically. For example, when the renal protective agent is a metal scavenger, it is used at a dose of 500 to 2000 mg, preferably 700 to 1500 mg, more preferably 800 to 1200 mg, per day for adults. As a more specific example, when Ca-DTPA or Zn-DTPA is used as a metal scavenger in combination, the dose is particularly preferably 900 to 1100 mg per day for adults. When the renal protective agent is a diuretic, it is used at a dose of 10 to 200 mg, preferably 20 to 150 mg, more preferably 40 to 100 mg, per day for adults. As a more specific example, when furosemide is used as a diuretic in combination, the dose is particularly preferably 40 to 80 mg per day for adults, and when spironolactone or eplerenone is used as a diuretic in combination, the dose is particularly preferably 50 to 100 mg per day for adults.

The dosage form of the $^{225}$Ac-labeled anti-MUC5AC humanized antibody as an active ingredient in the radioactive antitumor agent 1 of the present invention and the renal protective agent to be administered in combination is not particularly limited, and when administering the $^{225}$Ac-labeled anti-MUC5AC humanized antibody, it is sufficient that the renal toxicity caused by the administration of a drug containing Actinium-225 or from Actinium-225 or daughter nuclides of Actinium-225 is reduced. The administration form of the $^{225}$Ac-labeled anti-MUC5AC humanized antibody as the active ingredient in the radioactive antitumor agent 1 of the present invention includes oral administration and parenteral administration such as intravenous administration, subcutaneous administration, intraperitoneal administration, intramuscular administration, and the like. Parenteral administration such as intravenous administration, subcutaneous administration, intraperitoneal administration, intramuscular administration, and the like is preferred, and intravenous administration is more preferred.

In addition, the administration form of the renal protective agent as the active ingredient of the radioactive antitumor agent 1 of the present invention includes oral administration and parenteral administration such as intravenous administration, subcutaneous administration, intraperitoneal administration, intramuscular administration and the like, and is preferably an administration form in which these renal protective agents are clinically used, and more preferably selected according to the type of the aforementioned renal protective agent. For example, when the renal protective agent is an angiotensin II receptor antagonist, angiotensin converting enzyme inhibitor, Ca antagonist, diuretic, $\alpha$-blocker, or $\beta$-blocker, oral administration is preferred, and when the renal protective agent is a metal scavenger, parenteral administration such as intravenous administration, subcutaneous administration, intraperitoneal administration, intramuscular administration and the like is preferred, and intravenous administration, subcutaneous administration or intraperitoneal administration is more preferred. Similarly, the dosage form of the $^{225}$Ac-labeled anti-MUC5AC humanized antibody and the renal protective agent as active ingredients in the radioactive antitumor agent 2 of the present invention are not particularly limited, and when administering the $^{225}$Ac-labeled anti-MUC5AC humanized antibody, it is sufficient that the renal toxicity caused by the administration of a drug containing Actinium-225 or from Actinium-225 or daughter nuclides of Actinium-225 is reduced. The administration form of the $^{225}$Ac-labeled anti-MUC5AC humanized antibody and the renal protective agent as the active ingredients in the radioactive antitumor agent 2 of the present invention includes oral administration and parenteral administration such as intravenous administration, subcutaneous administration, intraperitoneal administration, intramuscular administration, and the like. Parenteral administration such as intravenous administration, subcutaneous administration, intraperitoneal administration, intramuscular administration, and the like is preferred, and intravenous administration is more preferred.

The administration form of the radioactive antitumor agent 1 of the present invention or the radioactive antitumor agent 2 of the present invention includes, for example,

(1) administration as a single preparation obtained by simultaneously formulating a $^{225}$Ac-labeled anti-MUC5AC humanized antibody and a renal protective agent,
(2) simultaneous administration of two preparations obtained by separately formulating a $^{225}$Ac-labeled anti-MUC5AC humanized antibody and a renal protective agent by the same administration route,
(3) administration of two preparations obtained by separately formulating a $^{225}$Ac-labeled anti-MUC5AC humanized antibody and a renal protective agent via the same administration route at different times,
(4) simultaneous administration of two preparations obtained by separately formulating a $^{225}$Ac-labeled anti-MUC5AC humanized antibody and a renal protective agent by different administration routes,
(5) administration of two preparations obtained by separately formulating a $^{225}$Ac-labeled anti-MUC5AC humanized antibody and a renal protective agent by different administration routes at different times,

and the like.

From the aspects of convenience, administration as a single preparation or simultaneous administration of two

preparations by the same route is preferred. From the aspects of maximizing the renal toxicity-reducing effect of renal protective agents, administration of two types of preparations by the same administration route at different times, or administration of two types of preparations by different administration routes at different times is also a preferred embodiment.

The aforementioned administration at different times includes both administration of the $^{225}$Ac-labeled anti-MUC5AC humanized antibody before administration of the renal protective agent, and administration of the $^{225}$Ac-labeled anti-MUC5AC humanized antibody after administration of the renal protective agent.

Formulation can be performed, for example, by dissolving the conjugate of the present invention and/or a renal protective agent in a solvent mainly composed of water and approximately isotonic with the living body. Where necessary, other pharmaceutically acceptable components may also be contained.

In the present invention, when simply referred to a "radioactive antitumor agent", it is intended to include the radioactive antitumor agents 1 and 2 of the present invention, and it includes both a single preparation obtained by simultaneously formulating a $^{225}$Ac-labeled anti-MUC5AC humanized antibody and a renal protective agent, and each of two types of preparations obtained by separately formulating a $^{225}$Ac-labeled anti-MUC5AC humanized antibody and a renal protective agent.

The radioactive antitumor agent of the present invention can contain any additives, such as a pharmaceutically acceptable carrier, in addition to the $^{225}$Ac-labeled anti-MUC5AC humanized antibody and a renal protective agent as active ingredients. Examples of the pharmaceutically acceptable carrier include, but are not limited to, excipients such as sucrose, starch, mannit, sorbit, lactose, glucose, cellulose, talc, calcium phosphate, calcium carbonate, and the like, binders such as cellulose, methylcellulose, hydroxypropylcellulose, polypropyl pyrrolidone, gelatin, gum arabic, polyethylene glycol, sucrose, starch, and the like, disintegrants such as starch, carboxymethylcellulose, hydroxypropylstarch, sodium-glycol-starch, sodium hydrogen carbonate, calcium phosphate, calcium citrate, and the like, lubricants such as magnesium stearate, aerogel, talc, sodium lauryl sulfate, and the like, aromatics such as citric acid, menthol, glycyl lysine ·ammonium salt, glycine, orange powder, and the like, preservatives such as sodium benzoate, sodium bisulfite, methylparaben, propylparaben, and the like, stabilizers such as citric acid, sodium citrate, acetic acid, and the like, suspending agents such as methylcellulose, polyvinylpyrrolidone, aluminum stearate, and the like, dispersing agents such as surfactant and the like, diluents such as water, saline, orange juice, and the like, base waxes such as cacao butter, polyethylene glycol, white kerosene, and the like, and the like.

In one embodiment, the radioactive antitumor agent of the present invention may be formulated as a preparation suitable for oral administration. A preparation suitable for oral administration includes liquid agents in which an effective amount of a substance is dissolved in a diluent such as water or physiological saline, capsule, granule, powder or tablet containing an effective amount of a substance as a solid or granule, suspensions containing an effective amount of a substance suspended in a suitable dispersion medium, emulsions in which a solution in which an effective amount of a substance is dissolved is dispersed and emulsified in a suitable dispersion medium, and the like.

The administration route of the $^{225}$Ac-labeled anti-MUC5AC humanized antibody as an active ingredient in the radioactive antitumor agent 1 of the present invention and the renal protective agent to be administered in combination is not particularly limited, and when administering the $^{225}$Ac-labeled anti-MUC5AC humanized antibody, it is sufficient that the renal toxicity caused by the administration of a drug containing Actinium-225 or from Actinium-225 or daughter nuclides of Actinium-225 is reduced. Similarly, the administration route of the $^{225}$Ac-labeled anti-MUC5AC humanized antibody and the renal protective agent as active ingredients in the radioactive antitumor agent 2 of the present invention is not particularly limited, and when administering the $^{225}$Ac-labeled anti-MUC5AC humanized antibody, it is sufficient that the renal toxicity caused by the administration of a drug containing Actinium-225 or from Actinium-225 or daughter nuclides of Actinium-225 is reduced. Examples of the administration route include (1) intravenous injection of $^{225}$Ac-labeled anti-MUC5AC humanized antibody, and oral administration of renal protective agent, (2) intravenous injection of both $^{225}$Ac-labeled anti-MUC5AC humanized antibody and renal protective agent, and the like.

In another embodiment, the radioactive antitumor agent of the present invention may be formulated as a preparation suitable for parenteral administration. A preparation suitable for parenteral administration (e.g., intravenous injection, subcutaneous injection, muscle injection, topical injection, and the like) include aqueous and non-aqueous isotonic sterile injection liquids, which may contain antioxidant, buffer, bacteriostatic agent, isotonicity agent, and the like. In addition, aqueous and non-aqueous sterile suspensions can be mentioned, which may contain suspending agent, solubilizer, thickener, stabilizer, antiseptic, and the like. The preparation can be packaged in unit dose or multiple doses in containers such as ampoules and vials. Alternatively, the active ingredient and the pharmaceutically acceptable carrier can also be freeze-dried and stored in a state where they can be dissolved or suspended in a suitable sterile vehicle immediately before use.

The radioactive antitumor agent of the present invention using a $^{225}$Ac-labeled anti-MUC5AC humanized antibody and a renal protective agent as active ingredients has an antitumor effect on mammals (e.g., human, mouse, rat, monkey, guinea pig, chimpanzee, sheep, goat, dog, cat, pig, cow, horse), preferably human, and therefore is useful for the treatment of various types of cancer.

Preferred target diseases include, for example, pancreatic cancer, thyroid cancer, liver cancer, colorectal cancer, gastric cancer, urothelial cancer, breast cancer, cervical cancer, ovarian cancer, and endometrial carcinoma, and particularly, application to pancreatic cancer is preferred.
Examples of the cancer to be treated by the present invention also include bile duct cancer.
There are plural reports stating that MUC5AC is an antigen carrier for CA19-9 (PLoS ONE (December 2011, Volume 6, Issue 12, e29180, p1-10)). Therefore, examples of the cancer to be treated by the present invention also include biliary tract cancer, uterine cancer, lung cancer, and esophageal cancer overexpressing CA19-9, and these can be treated efficiency.
According to the embodiments of the present invention described above, a radioactive antitumor agent is provided that is superior in the specificity for MUC5AC and accumulation in tumor and has reduced renal toxicity.
The above-mentioned embodiment of the present invention includes the following technical ideas.

[1] A radioactive antitumor agent comprising a $^{225}$Ac-labeled anti-MUC5AC humanized antibody (humanized antibody labeled with actinium-225 that specifically binds to mucin subtype 5AC) as an active ingredient, which is used in combination with a drug for reducing renal toxicity.
[2] A radioactive antitumor agent comprising a $^{225}$Ac-labeled anti-MUC5AC humanized antibody (humanized antibody labeled with actinium-225 that specifically binds to mucin subtype 5AC) and a drug for reducing renal toxicity as active ingredients.
[3] The radioactive antitumor agent of the above-mentioned [1] or [2], wherein the drug for reducing renal toxicity is a drug for reducing renal toxicity caused by the administration of a drug containing Actinium-225.
[4] The radioactive antitumor agent of the above-mentioned [1] or [2], wherein the drug for reducing renal toxicity is a drug for reducing renal toxicity caused by Actinium-225 or a daughter nuclide of Actinium-225.
[5] The radioactive antitumor agent of any of the above-mentioned [1] to [4], wherein the aforementioned renal toxicity is late renal toxicity that occurs after a lapse of not less than 10 weeks after administration of the aforementioned radioactive antitumor agent.
[6] The radioactive antitumor agent of any of the above-mentioned [1] to [5], wherein the aforementioned drug for reducing renal toxicity is a diuretic or a metal scavenger.
[7] The radioactive antitumor agent of the above-mentioned [6], wherein the aforementioned metal scavenger comprises DTPA, Ca-DTPA, or Zn-DTPA.
[8] The radioactive antitumor agent of the above-mentioned [7], wherein the aforementioned metal scavenger comprises Zn-DTPA.
[9] The radioactive antitumor agent of the above-mentioned [6], wherein the aforementioned diuretic comprises furosemide, spironolactone, or eplerenone.
[10] The radioactive antitumor agent of the above-mentioned [9], wherein the aforementioned diuretic comprises furosemide.
[11] The radioactive antitumor agent of any of the above-mentioned [1] to [10], wherein the aforementioned anti-MUC5AC humanized antibody comprises a heavy chain variable region consisting of

(1) the amino acid sequence shown in SEQ ID NO: 1 (H01), an amino acid sequence having not less than 90% or not less than 95% sequence identity with the amino acid sequence shown in SEQ ID NO: 1, or an amino acid sequence shown in SEQ ID NO: 1 wherein 3, 2 or 1 amino acid is/are deleted, substituted or added,
(2) the amino acid sequence shown in SEQ ID NO: 2 (H02), an amino acid sequence having not less than 90% or not less than 95% sequence identity with the amino acid sequence shown in SEQ ID NO: 2, or an amino acid sequence shown in SEQ ID NO: 2 wherein 3, 2 or 1 amino acid is/are deleted, substituted or added,
(3) the amino acid sequence shown in SEQ ID NO: 3 (H03), an amino acid sequence having not less than 90% or not less than 95% sequence identity with the amino acid sequence shown in SEQ ID NO: 3, or an amino acid sequence shown in SEQ ID NO: 3 wherein 3, 2 or 1 amino acid is/are deleted, substituted or added, or
(4) the amino acid sequence shown in SEQ ID NO: 4 (H04), an amino acid sequence having not less than 90% or not less than 95% sequence identity with the amino acid sequence shown in SEQ ID NO: 4, or an amino acid sequence shown in SEQ ID NO: 4 wherein 3, 2 or 1 amino acid is/are deleted, substituted or added, and a light chain variable region consisting of
(5) the amino acid sequence shown in SEQ ID NO: 5 (L01), an amino acid sequence having not less than 90% or not less than 95% sequence identity with the amino acid sequence shown in SEQ ID NO: 5, or an amino acid sequence shown in SEQ ID NO: 5 wherein 3, 2 or 1 amino acid is/are deleted, substituted or added,
(6) the amino acid sequence shown in SEQ ID NO: 6 (L02), an amino acid sequence having not less than 90% or not less than 95% sequence identity with the amino acid sequence shown in SEQ ID NO: 6, or an amino acid sequence shown in SEQ ID NO: 6 wherein 3, 2 or 1 amino acid is/are deleted, substituted or added,
(7) the amino acid sequence shown in SEQ ID NO: 7 (L03), an amino acid sequence having not less than 90%

or not less than 95% sequence identity with the amino acid sequence shown in SEQ ID NO: 7, or an amino acid sequence shown in SEQ ID NO: 7 wherein 3, 2 or 1 amino acid is/are deleted, substituted or added, or
(8) the amino acid sequence shown in SEQ ID NO: 8 (L04), an amino acid sequence having not less than 90% or not less than 95% sequence identity with the amino acid sequence shown in SEQ ID NO: 8, or an amino acid sequence shown in SEQ ID NO: 8 wherein 3, 2 or 1 amino acid is/are deleted, substituted or added.

[12] The radioactive antitumor agent of any of the above-mentioned [1] to [11], wherein the aforementioned anti-MUC5AC humanized antibody is a humanized antibody having

(1) a heavy chain variable region consisting of the amino acid sequence shown by SEQ ID NO: 1 (H01) or an amino acid sequence having 95% or more sequence identity with the amino acid sequence shown by SEQ ID NO: 1, and
(7) a light chain variable region consisting of the amino acid sequence shown by SEQ ID NO: 7 (L03) or an amino acid sequence having 95% or more sequence identity with the amino acid sequence shown by SEQ ID NO: 7.

[13] The radioactive antitumor agent of any of the above-mentioned [1] to [12], wherein the aforementioned $^{225}$Ac-labeled anti-MUC5AC humanized antibody is a conjugate of a chelating agent complexed with actinium-225 and the aforementioned anti-MUC5AC humanized antibody, and the Fc region of the aforementioned anti-MUC5AC humanized antibody is site-specifically modified with the aforementioned chelating agent via a linker.
[14] The radioactive antitumor agent of the above-mentioned [11], wherein the aforementioned linker comprises a peptide represented by the following formula (i) which consists of not less than 13 and not more than 17 amino acid residues, and which is formed by a crosslinking reaction of the aforementioned peptide modified by a crosslinking agent and the aforementioned antibody:

(Xa)-Xaa1-(Xb)-Xaa2-(Xc)-Xaa3-(Xd)··· (i)

wherein Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa1 and Xaa3 are linked, and
Xaa2 is a lysine residue, an arginine residue, a cysteine residue, an aspartic acid residue, a glutamic acid residue, 2-aminosuberic acid, or diamino propionic acid, and modified with the aforementioned crosslinking agent.

[15] The radioactive antitumor agent of the above-mentioned [13] or [14], wherein the aforementioned chelating agent has a structure derived from a compound represented by the following formula (A) or a salt thereof:

(A)

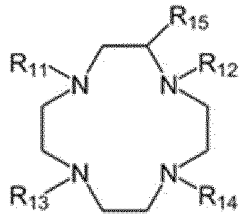

wherein in the formula (A), $R_{11}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating

with the antibody, p is an integer of not less than 0 and not more than 3, $R_{15}$ is a hydrogen atom when $R_{12}$ is a substituent for conjugating with the antibody, and $R_{15}$ is a substituent for conjugating with the antibody when $R_{12}$ is not a substituent for conjugating with the antibody.

[16] The radioactive antitumor agent of any of the above-mentioned [1] to [15], which is used to treat pancreatic cancer, thyroid cancer, liver cancer, colorectal cancer, stomach cancer, urothelial cancer, breast cancer, cervical cancer, ovarian cancer, endometrial cancer or bile duct cancer.

[17] A drug for reducing renal toxicity, which is used in combination with a radioactive antitumor agent comprising $^{225}$Ac-labeled anti-MUC5AC humanized antibody as an active ingredient.

[18] The drug of the above-mentioned [17], wherein the drug for reducing renal toxicity is a diuretic or a metal scavenger.

[19] A combination drug comprising a radioactive antitumor agent comprising a $^{225}$Ac-labeled anti-MUC5AC humanized antibody as an active ingredient, and a drug for reducing renal toxicity.

[20] A method for treating a tumor, comprising administering an effective amount of a $^{225}$Ac-labeled anti-MUC5AC humanized antibody and an effective amount of a drug for reducing renal toxicity in combination to a mammal.

[21] Use of a $^{225}$Ac-labeled anti-MUC5AC humanized antibody in the production of a radioactive antitumor agent to be used in combination with a drug for reducing renal toxicity.

[22] Use of a $^{225}$Ac-labeled anti-MUC5AC humanized antibody and a drug for reducing renal toxicity in the production of a radioactive antitumor agent.

[23] A $^{225}$Ac-labeled anti-MUC5AC humanized antibody for treating a tumor, which is used in combination with a drug for reducing renal toxicity.

[24] A combination of a $^{225}$Ac-labeled anti-MUC5AC humanized antibody for treating a tumor and a drug for reducing renal toxicity.

[25] A kit for producing a radioactive antitumor agent to be used in combination with a drug for reducing renal toxicity, comprising a chelating agent complexed with Actinium-225 and an anti-MUC5AC humanized antibody, wherein the aforementioned anti-MUC5AC humanized antibody is the antibody of the above-mentioned [11] or [12].

[26] A kit for producing a radioactive antitumor agent, comprising a chelating agent complexed with Actinium-225, an anti-MUC5AC humanized antibody, and a drug for reducing renal toxicity, wherein the aforementioned anti-MUC5AC humanized antibody is the antibody of the above-mentioned [11] or [12].

[27] A kit for treating cancer, comprising a drug for reducing renal toxicity, a chelating agent complexed with Actinium-225, and an anti-MUC5AC humanized antibody, wherein the aforementioned anti-MUC5AC humanized antibody is the antibody of the above-mentioned [11] or [12].

[28] A kit for use in RI internal therapy for cancer, comprising a drug for reducing renal toxicity, a chelating agent complexed with Actinium-225, and an anti-MUC5AC humanized antibody, wherein the aforementioned anti-MUC5AC humanized antibody is the antibody of the above-mentioned [11] or [12].

According to the radioactive antitumor agent of the present invention, since a conjugate containing a humanized antibody that specifically binds to MUC5AC and Actinium-225 that emits $\alpha$-rays is contained as an active ingredient, when used for RI internal therapy of cancer, it accumulates specifically in tumor expressing MUC5AC and can irradiate $\alpha$-rays specifically on tumor cells without affecting normal cells, whereby higher safety and higher therapeutic effect is obtained. Furthermore, since it is used in combination with a drug for reducing renal toxicity, side effects feared in cancer therapy are reduced.

The present invention is explained in detail in the following by referring to Examples and the like. The present invention is not limited thereto.

[Example]

Production Example 1. Production of anti-MUC5AC humanized antibody

The amino acid sequences of various variable regions to which a signal sequence was added and the amino acid sequences of various constant regions were converted into base sequences while considering codon usage suitable for expression in CHO cells. A Kozak sequence was added to the initiation codon site of the signal sequence, and a stop codon was added to the C-terminal side of the constant region. Furthermore, restriction enzyme sites were added to the upstream of the Kozak sequence and downstream of the stop codon so that they could be introduced into the expression gene transfer site of a mammalian cell expression plasmid (pcDNA3.4). Each DNA fragment designed in this way was prepared by chemical synthesis. A DNA fragment containing a variable region to be a desired H chain and a desired L chain and a DNA fragment containing a constant region were ligated by fusion PCR.

The prepared various antibody genes were subjected to restriction enzyme treatment and then purified. Similarly, a mammalian cells transient expression plasmid (pcDNA3.4) was also treated with the same restriction enzyme and

then purified. The both fragments were mixed at an appropriate mixing ratio and ligated. The ligation reaction solution was mixed with Escherichia coli DH5α competent cells to perform transformation. The resulting transformants were subjected to colony PCR, single colony isolation, plasmid extraction from small-scale culture medium, and nucleotide sequencing of the insert portion were performed. A plasmid (Escherichia coli clone) into which the full-length designed antibody gene was correctly inserted in the intended direction with the designed sequence (Escherichia coli clone) was selected. The selected Escherichia coli clone was subjected to large scale culture, and plasmid extraction and purification including an endotoxin removal step were performed. The concentration of the purified plasmid was calculated by measuring the absorbance at 260 nm.

Transient expression by CHO cells was performed using the ExpiCHO System (manufactured by Thermo Fisher Scientific). From each of the prepared H chain expression plasmids and each of the prepared L chain expression plasmid, one H chain and one L chain were selected to achieve the desired combination, transfected by the lipofection method, cultured, and fed. After 7 days - 13 days from transfection, the culture medium was recovered. The culture supernatants after centrifugation and filtration were added to Protein A column and the antibody was purified by general affinity column chromatography (washing after adsorption, elution with acidic buffer, neutralization of eluate). The concentration of the purified antibody was calculated by measuring the absorbance at 280 nm.

The following anti-MUC5AC humanized antibodies were prepared using the method described above. The antibody numbers assigned to the combinations of the heavy chain variable region and the light chain variable region are shown below.

antibody 1: H01L03
antibody 2: H01L04
antibody 3: H02L04
antibody 4: H04L04

As used herein, H01, H02 and H04 are heavy chain variable regions respectively shown in SEQ ID NO: 1, SEQ ID NO: 2 and SEQ ID NO: 4, L03 and L04 are light chain variable regions respectively shown in SEQ ID NO: 7 and SEQ ID NO: 8. The antibodies used in the following Examples were combinations of heavy chain constant region 1 (SEQ ID NO: 25) and light chain constant region 1 (SEQ ID NO: 26), and the heavy chain variable region and light chain variable region of the above-mentioned antibody 1 to antibody 4.

Production Example 2. Site-specific antibody modification by peptide linker

(1) Antibody modification step

An antibody-modification peptide was produced by the method described in WO 2017/217347 to obtain a peptide containing 17 amino acid residues represented by the following formula (P3). The amino acid sequence of this peptide was the same as the sequence in which Xaa2 of SEQ ID NO: 10 was a lysine residue, and the side chain terminal amino group of the lysine residue was modified with the structure shown by $R_1$. In addition, two cysteine residues form a disulfide bond with each other, and to the N-terminal of the peptide is added ethyl azide as an atomic group containing an azide group, which is the second atomic group, via a linker structure having diglycolic acid and eight PEGs.

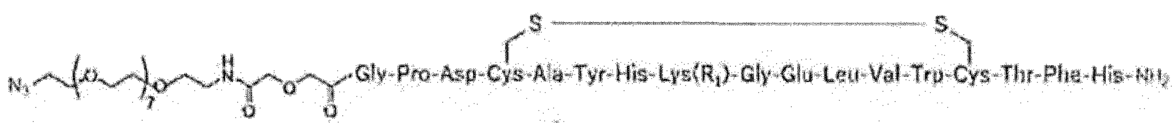

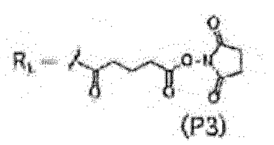

(P3)

wherein in the formula (P3), Gly is glycine, Pro is proline, Asp is aspartic acid, Cys is cysteine, Ala is alanine, Tyr is tyrosine, His is histidine, Lys is lysine, Glu is glutamic acid, Leu is leucine, Val is valine, Trp is tryptophan, Thr is threonine, and Phe is phenylalanine, respectively.

A mixture of the peptide and an anti-MUC5AC humanized antibody (antibody 1) produced in Production Example 1 in a sodium acetate buffer (pH 6.0) was reacted at room temperature for 30 min to give a solution containing a peptide-modified antibody. The peptide-modified antibody has an Fc region of the antibody site-specifically modified by the

above-mentioned peptide.

(2) Peptide-modified antibody separation step

The peptide-modified antibody was diluted with 1 mol/L sodium acetate buffer (pH 6.0), added to Protein A column (manufactured by GE Healthcare, HiTrap MabSelect SuRe), and a 0.05 mol/L sodium acetate buffer (pH 5.7) containing 0.15 mol/L sodium chloride was flown. A peptide-modified antibody (hereinafter to be also referred to as "divalent antibody") to which two peptide molecules modify was recovered, and the concentration was adjusted such that the concentration of the divalent antibody contained in the recovered fraction was 15 mg/mL. Thereafter, 0.05 mol/L sodium acetate buffer (pH 3.5) containing 0.15 mol/L sodium chloride was flown into Protein A column, a peptide-modified antibody (hereinafter to be also referred to as "monovalent antibody") to which one molecule of peptide modify was recovered, and the concentration was adjusted such that the concentration of the monovalent antibody contained in the recovered fraction was 15 mg/mL.

Production Example 3. Production of $^{225}$Ac-labeled anti-MUC5AC humanized antibody

(1) Chelating agent synthesis step

The structure of the chelate site (DOTAGA-DBCO) used in this Example is shown in the following formula (L1-5). DOTAGA-DBCO shown in the formula (L1-5) was produced according to the method described in Bernhard et al. DOTAGA-Anhydride: A Valuable Building Block for the Preparation of DOTA-Like Chelating Agents Chem. Eur. J. 2012, 18, 7834-7841. This chelate site was dispersed in 0.1 mol/L sodium acetate buffer (pH 6.0) as a solvent to give a dispersion containing 1.7 mmol/L chelate site. A mixture of this dispersion (0.004 mL), a $^{225}$Ac ion-containing solution (0.2 mol/L aqueous hydrochloric acid solution, radioactivity concentration 1225 MBq/mL, prepared from one manufactured by Oak Ridge National Laboratory, liquid amount 0.004 mL) 4.9 MBq (calculated by attenuation from the amount of radioactivity at test date and time) as a radioactive metal source, and 0.1 mol/L sodium acetate buffer (pH 6.0, 0.06 mL) was reacted under heating conditions to give a $^{225}$Ac complex solution. The molar ratio of the chelate site and the radioactive metal ion was chelate site:$^{225}$Acion = about 670:1, the heating temperature of the reaction mixture was set to 70°C, and the heating time was set to 30 min.

L1-5

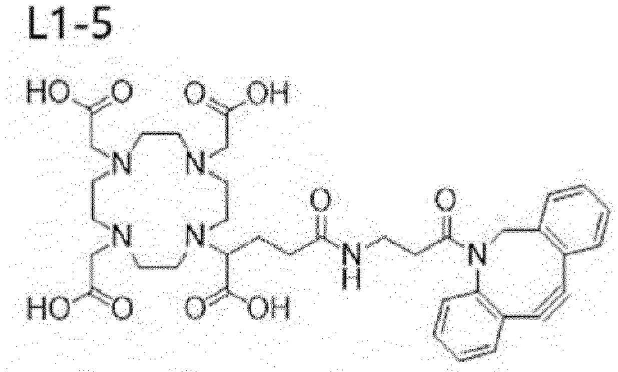

DOTAGA-DBCO

The radiochemical purity of the obtained $^{225}$Ac complex was measured by the following method. That is, a part of the $^{225}$Ac complex solution was developed by thin layer chromatography (manufactured by Agilent, model number: SGI0001, developing solvent: acetonitrile/water mixed solution (volume ratio 1:1)), and then measured by radio $\gamma$-TLC Analyzer (manufactured by raytest, MODEL GITA Star). The percentage of the radioactivity (count) of the peak detected near the origin with respect to the detected total radioactivity (count) was defined as the radiochemical purity (%) of the $^{225}$Ac complex. As a result, the radiochemical purity of the $^{225}$Ac complex was 85%. The obtained $^{225}$Ac complex solution was directly used for the labeling step.

(2) Labeling step

The eluate of the monovalent antibody obtained in Production Example 2, and the solution of the $^{225}$Ac complex obtained in the aforementioned step (1) were each added to 0.02 mol/L (20 mM) ascorbic acid-containing 0.09 mol/L sodium acetate buffer, and click reacted at 37°C for 120 min to give an $^{225}$Ac-labeled monovalent antibody. The amount

of the $^{225}$Ac complex and the amount of the peptide-modified antibody were 44 μmol and 46 pmol, respectively, and the molar ratio of the first atomic group (DBCO) and the second atomic group (azide) was about 1:1.

Furthermore, a solution of the $^{225}$Ac-labeled monovalent antibody obtained by reacting at 37°C for 2 hr was purified using ultrafiltration filter (manufactured by Merck, model number: UFC505096), and subjected to the subsequent experiments. The radiochemical purity of the $^{225}$Ac-labeled monovalent antibody after purification (amount of radioactivity calculated by attenuation from the amount of radioactivity at test date and time: 0.303 MBq) was 93%, and the radiochemical yield was 39%. As used herein, the radiochemical purity is the ratio (%) of the radioactivity count of the peak corresponding to the $^{225}$Ac-labeled monovalent antibody to the total radioactivity count of the thin layer plate when analyzed by thin layer chromatography, and the radiochemical yield is the ratio (%) of the radioactivity amount calculated from the radioactivity count of the $^{225}$Ac-labeled monovalent antibody to the radioactivity amount calculated from the radioactivity count at the start of the labeling process measured by γ-ray spectrometer (Ge semiconductor detector: GMX10P4-70 (manufactured by ORTEC), Multi Channel Analyzer: M7-000 (manufactured by SEIKO EG&G), data processing: Spectrum Navigator: DS-P300 (manufactured by SEIKO EG&G) and Gamma Studio: DS-P600 (manufactured by SEIKO EG&G)).

Examples 1 to 7, Comparative Examples 1 to 5: Reduction of delayed renal toxicity by combined administration of compound_ expected to have renal protective effect

Whether combined administration of a $^{225}$Ac-labeled anti-MUC5AC humanized antibody prepared using $^{225}$Ac-labeled DOTAGA-DBCO and a compound expected to have a renal protective effect (renal protective agent) reduces delayed renal toxicity caused by radiation emitted from $^{225}$Ac or $^{225}$Ac daughter nuclide was evaluated.

The $^{225}$Ac-labeled anti-MUC5AC humanized antibody produced in Production Example 3 was administered to CD-1 mice (5 weeks old, male, manufactured by Charles River Japan), each group n=5 at a dose of 15 kBq/mouse and, as a renal protective agent, any of Zn-DTPA, furosemide, spironolactone, and eplerenone was administered in combination at the dosage, administration method, administration frequency, and initial administration timing shown in Table 1. That is, a group to which only $^{225}$Ac-labeled anti-MUC5AC humanized antibody was administered (Comparative Example 1), a group to which $^{225}$Ac-labeled anti-MUC5AC humanized antibody and each renal protective agent administered in combination (Example 1 to Example 7), and a group to which only a renal protective agent is administered without administration of $^{225}$Ac-labeled anti-MUC5AC humanized antibody (Comparative Examples 2 to 5) were established. The dose of the $^{225}$Ac-labeled anti-MUC5AC humanized antibody, each renal protective agent, and feeding conditions are shown in Table 1. The sustained release tablets used for the transdermal administration group of each renal protective agent were those manufactured by Innovative Research of America, and the mixed feed containing 0.004% of each renal protective agent used was manufactured by Oriental Yeast Co., Ltd.

Each individual was observed for 26 weeks (184 days) from the day of administration of the $^{225}$Ac-labeled anti-MUC5AC humanized antibody. During the observation period, body weight measurement, blood cell measurement, and renal toxicity marker measurement were performed over time and signs of toxicity were evaluated.

[Table 1]

| | $^{225}Ac$-labeled antibody | renal protective agent | | | | | feeding conditions |
|---|---|---|---|---|---|---|---|
| | | name | dose | administration method | administration frequency | timing of initial administration | |
| Example 1 | 15 kBq/mouse | Zn-DTPA | 3.20 mg/mouse | intraperitoneal | once/day (continued for 7 days) | after administration of labeled antibody | normal feed |
| Example 2 | | furosemide | 0.33 mg/mouse | oral | | 2 hr before administration of labeled antibody | |
| Example 3 | | spironolactone | 0.26 mg/mouse | | | | mixed feed (containing 0.004% renal protective agent) after day 8 |
| Example 4 | | eplerenone | 0.25 mg/mouse | | | | |
| Example 5 | | furosemide | 0.17 mg/day | transdermal | continued during breeding period | sustained release tablet was subcutaneously implanted on previous day of labeled antibody administration (reimplanted every 60 days) | normal feed |
| Example 6 | | spironolactone | | | | | |
| Example 7 | | eplerenone | | | | | |
| Comparative Example 1 | | - | - | - | - | - | |
| Comparative Example 2 | - | Zn-DTPA | 3.20 mg/mouse | intraperitoneal | once/day (continued for 7 days) | after medium administration | |
| Comparative Example 3 | | furosemide | 0.17 mg/day | transdermal | continued during breeding period | sustained release tablet was subcutaneously implanted on previous day of medium administration (reimplanted every 60 days) | |
| Comparative Example 4 | | spironolactone | | | | | |
| Comparative Example 5 | | eplerenone | | | | | |

[Evaluation 1] Body weight transition

The average body weight transition of each group during the observation period from the day of administration of the $^{225}$Ac-labeled anti-MUC5AC humanized antibody are shown in Fig. 1 to Fig. 4. A tendency toward decrease in the average body weight was not observed in any group.

[Evaluation 2] Urinary renal toxicity marker

Urine was collected from each individual 16 weeks after the administration of the $^{225}$Ac-labeled anti-MUC5AC humanized antibody, and neutrophil gelatinase-associated lipocalin (NGAL), and creatinine concentration of the urine samples were measured by ELISA. NGAL levels are almost undetectable in normal kidney. However, when renal damage occurs, it is rapidly induced from the distal renal tubule, as well as reabsorption from proximal renal tubule is inhibited and the rate of excretion into the urine increases. Thus, it is used as a marker for renal tubular damage. The NGAL value corrected for urine concentration was calculated by dividing the obtained NGAL value by the creatinine value (urinary NGAL/creatinine ratio, hereinafter referred to as urinary N/C ratio). The calculated urinary N/C ratio was confirmed using Stat PreClinica (manufactured by Takumi Information Technology Co., Ltd.) for the homoscedasticity of the measured values. Since homoscedasticity was confirmed for all items, a significant difference test was performed using the Dunnett method, which is a parametric test, using the $^{225}$Ac-labeled anti-MUC5AC humanized antibody administration group as a control group. The significance level was set to 5%.

The calculation results of urinary N/C ratio at 16 weeks from $^{225}$Ac-labeled anti-MUC5AC humanized antibody administration are shown in Table 2. Graphs of urinary N/C ratio of the intraperitoneal administration group (Example 1) and the oral administration group (Example 2) are shown in Fig. 5. At 16 weeks, the N/C ratio significantly decreased in the Zn-DTPA combined administration group (Example 1) as compared with the $^{225}$Ac-labeled anti-MUC5AC humanized antibody administration group (Comparative Example 1). Among other groups, all combined administration groups that received these compounds orally oral all compounds combined administration groups (Examples 2 to 4) and transdermal furosemide combined administration group (Example 5) did not show a statistically significant difference, but showed values less than half as compared with the mean N/C ratio observed in Comparative Example 1. These results suggest that oral administration of Zn-DTPA, furosemide, spironolactone, or eplerenone may reduce renal tubular disorder caused by the administration of $^{225}$Ac-labeled anti-MUC5AC humanized antibody.

[Table 2]

| | N/C ratio |
|---|---|
| Example 1 | $0.2\pm0.17$ |
| Example 2 | $0.3\pm0.09$ |
| Example 3 | $0.9\pm0.41$ |
| Example 4 | $1.0\pm0.59$ |
| Example 5 | $1.7\pm0.72$ |
| Example 6 | $2.7\pm2.69$ |
| Example 7 | $10.6\pm4.94$ |
| Comparative Example 1 | $4.0\pm0.60$ |
| Comparative Example 2 | $1.1\pm0.60$ |
| Comparative Example 3 | $1.0\pm0.81$ |
| Comparative Example 4 | $0.8\pm0.67$ |
| Comparative Example 5 | $2.9\pm3.40$ |

[Evaluation 3] Autopsy and measurement of kidney weight

On the final day of the observation period (184 days from the day of administration of the $^{225}$Ac-labeled anti-MUC5AC humanized antibody), euthanasia and autopsy were performed by exsanguination under isoflurane anesthesia. After autopsy, the kidneys were collected and weighed. The obtained organ weight measurement results (excluding Example 7) were confirmed for the homoscedasticity of the measured values. Since homoscedasticity was confirmed

for all items, a significant difference test was performed using the Tukey method, which is a parametric test. The significance level was set to 5%.

As a result of autopsy, no external findings of toxicity were observed in any of the $^{225}Ac$-labeled anti-MUC5AC humanized antibody administration groups. Moreover, no significant difference was observed in kidney weight after autopsy in all groups (Table 3).

[Table 3]

| | body weight (g) | kidney weight (g) | kidney weight/body weight ratio |
|---|---|---|---|
| Example 1 | 41.97±3.1 | 0.58±0.11 | 0.014±0.002 |
| Example 2 | 41.30±4.8 | 0.57±0.11 | 0.014±0.001 |
| Example 3 | 39.35±4.5 | 0.53±0.09 | 0.013±0.001 |
| Example 4 | 39.06±2.0 | 0.53±0.07 | 0.013±0.001 |
| Example 5 | 44.48±3.6 | 0.67±0.15 | 0.015±0.003 |
| Example 6 | 40.97±3.4 | 0.52±0.07 | 0.013±0.001 |
| Example 7 | 42.06*1 | 0.53*1 | 0.013*1 |
| Comparative Example 1 | 43.14±3.0 | 0.69±0.14 | 0.016±0.003 |
| Comparative Example 2 | 44.48±4.5 | 0.75±0.12 | 0.017±0.001 |
| Comparative Example 3 | 42.68±2.3 | 0.78±0.06 | 0.018±0.000 |
| Comparative Example 4 | 43.90±3.5 | 0.66±0.05 | 0.015±0.001 |
| Comparative Example 5 | 46.29±5.6 | 0.83±0.12 | 0.018±0.001 |
| *1: standard deviation is not listed since n=2 at the time of autopsy | | | |

[Evaluation 4] Pathological evaluation

After organ weight measurement, the kidneys were fixed in 10% neutral buffered formalin solution for not less than one week. The fixed tissues were embedded in paraffin and sliced (section thickness: 3 pm), and the obtained tissue sections were stained with hematoxylin and eosin and subjected to pathological evaluation. The evaluation was performed by a person with professional qualification in toxicologic pathology.

The results of pathological evaluation of the kidney of each administration group are shown in Fig. 6. Representative pathological findings of degeneration/regeneration of tubular epithelial cells in the outer medulla of the kidney and basophilic tubules with inflammatory cell infiltration and fibrosis are respectively shown in Fig. 7 and Fig. 8. In Comparative Example 1, degeneration/regeneration of renal tubular epithelium in the outer medulla was observed in all five cases. In Comparative Example 1, diffuse changes were found in all cases (hereinafter referred to as "grade 2" in this paragraph). On the other hand, in Examples 1 to 7, changes of grade 2 or higher were found in 0 to 2 out of 5 cases. That is, in Examples 1 to 6, the frequency of occurrence of changes of grade 2 or higher in degeneration/regeneration of renal tubular epithelium in the outer medulla was lower than in Comparative Example 1.

In Comparative Example 1, basophilic tubules with inflammatory cell infiltration and fibrosis were observed in 3 out of 5 cases to the extent of spontaneous occurrence (hereinafter referred to as "grade 1" in this paragraph), and multifocally in 2 out of 5 cases (hereinafter referred to as "grade 2" in this paragraph). On the other hand, in Example 1, basophilic tubules with inflammatory cell infiltration and fibrosis were not observed in one case, grade 1 changes were observed in 3 cases, and grade 2 changes were observed in one case. in Examples 2, 5, and 7, the same change as grade 2 or higher was not observed. That is, in Examples 1, 2, 5 and 7, the incidence of occurrence of changes of grade 2 or higher in basophilic renal tubule with inflammatory cell infiltration and fibrosis was lower than in Comparative Example 1.

The above results indicate that all of the evaluated compounds may reduce degeneration/regeneration of the renal tubular epithelium in the outer medulla, which is a change suggesting damage to the renal tubular epithelium. Furthermore, in Zn-DTPA (Example 1) and furosemide (Examples 2 and 5), the results indicate that basophilic tubules with inflammatory cell infiltration and fibrosis may be reduced.

Production Example 4. Production of $^{225}$Ac-labeled anti-MUC5AC humanized antibody

A $^{225}$Ac-labeled anti-MUC5AC humanized antibody was obtained in the same manner as in Production Example 3 except that DOTAGA-DBCO was changed to DOTA-Bn-DBCO represented by the following structural formula. The radiochemical purity of the obtained purified $^{225}$Ac-labeled monovalent antibody (radioactivity amount: 1.1 MBq) was 95%, and the radiochemical yield thereof was 23%.

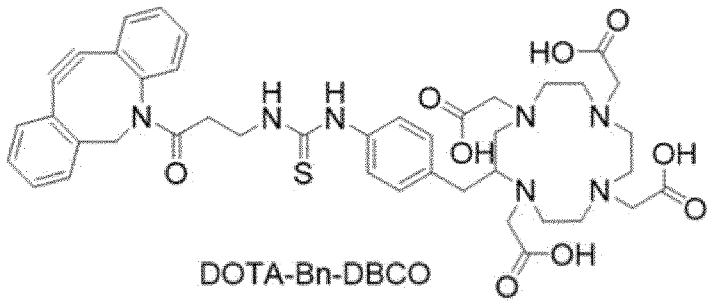

DOTA-Bn-DBCO

Example 8. Stability evaluation of $^{225}$Ac-labeled anti-MUC5AC humanized antibody

The $^{225}$Ac-labeled anti-MUC5AC humanized antibody produced according to Production Examples 3 and 4 was stored at room temperature or refrigerated for 2 weeks, and the radiochemical purity was evaluated each time point (0 day point, 1 day point, 2 day point, 7 day point, 14 day point). Note that 14 days from the end of production corresponds to about 1.5 half-lives with the physical half-life of $^{225}$Ac as the standard.

[Evaluation 5] Radiochemical purity

The radiochemical purity was analyzed by thin layer chromatography (TLC). The TLC conditions were similar to those used in Production Example 3 when radiochemical purity was examined. The results are shown in Table 4.

[Table 4]

| Production Example | storage temperature | radiochemical purity (%) | | | | |
|---|---|---|---|---|---|---|
| | | 0 day point | 1 day point | 2 day point | 7 day point | 14 day point |
| Production Example 3 | room temperature | 97 | 99 | 99 | 99 | 94 |
| | refrigerated | 96 | 99 | 98 | 99 | 95 |
| Production Example 4 | room temperature | 95 | 98 | 97 | 78 | 53 |
| | refrigerated | 95 | 98 | 98 | 92 | 89 |

The $^{225}$Ac-labeled anti-MUC5AC humanized antibody produced in Production Example 3, which had a structure derived from DOTAGA-DBCO, maintained a radiochemical purity of 99% or more when stored at room temperature for 7 days after production, and a radiochemical purity of 99% or more when stored under refrigeration for 7 days. It maintained a radiochemical purity of 90% or more when stored at room temperature for 14 days after production, and a radiochemical purity of 95% or more when stored under refrigeration for 14 days.

The $^{225}$Ac-labeled anti-MUC5AC humanized antibody produced in Production Example 4, which had a structure derived from DOTA-Bn-DBCO, maintained a radiochemical purity of 75% or more when stored at room temperature for 7 days after production, and a radiochemical purity of 90% or more when stored under refrigeration for 7 days. It maintained a radiochemical purity of 50% or more when stored at room temperature for 14 days after production, and a radiochemical purity of 85% or more when stored under refrigeration for 14 days.

From the above, it was considered that Zn-DTPA, furosemide, spironolactone, and eplerenone when administered in combination with the $^{225}$Ac-labeled anti-MUC5AC humanized antibody contribute to the reduction of renal tubular disorder and the improvement of pathological toxic findings that may occur late after a lapse of 10 weeks or more from the administration of the $^{225}$Ac-labeled anti-MUC5AC humanized antibody, and achieved the purpose of reducing renal toxicity associated with the administration of the $^{225}$Ac-labeled anti-MUC5AC humanized antibody.

Reference Example 1: Screening of humanized antibody using In-111 ($^{111}In$) -labeled antibody

Reference Example 1-1: Preparation of $^{111}In$-labeled antibody

To find an anti-MUC5AC antibody with high tumor accumulation ability and high maximum tolerated dose, various antibodies were labeled with $^{111}In$, administered to tumor-bearing mice, and SPECT-CT imaging was performed. Cumulative amount of radioactivity and absorbed dose in tumor and liver were calculated and compared using the obtained images.

The antibody used were 4 types of anti-MUC5AC humanized antibodies prepared in Production Example 1 and 1 type of anti-MUC5AC chimeric antibody disclosed in Patent Literature 1, and they were labeled with $^{111}In$.

The amino acid sequences of the heavy chain variable region and the light chain variable region (SEQ ID NOs: 23 and 24, respectively) of the chimeric antibody disclosed in Patent Literature 1 are as follows.

The $^{111}In$ labeling was performed on complexes of a labeling precursor having a structure represented by the following formula and various antibodies.

**[heavy chain variable region]**

E V K L V E S G G V L V K S G G S L K L S C A V S G F T F S N Y G M S W V R
Q T P E K R L E W V A T I S N S G R Y T Y F P D S V K G P F A I S R D N A K
N N L Y L Q M S S L R S A D T A L Y Y C T R H L D Y A N Y D A M D Y W G Q G
T S V T V S S

[light chain variable region]

D I V L T Q S P A S L A V S L G Q R A T I S C R A S K S V T T S D F S Y M H
W Y Q Q K P G Q P P K L L L Y L A S N L E S G V P D R F S G S G S G Y D F Y
L N I H P V E E E D A A T Y Y C Q H S R E F P W T F G G G T K L E I K

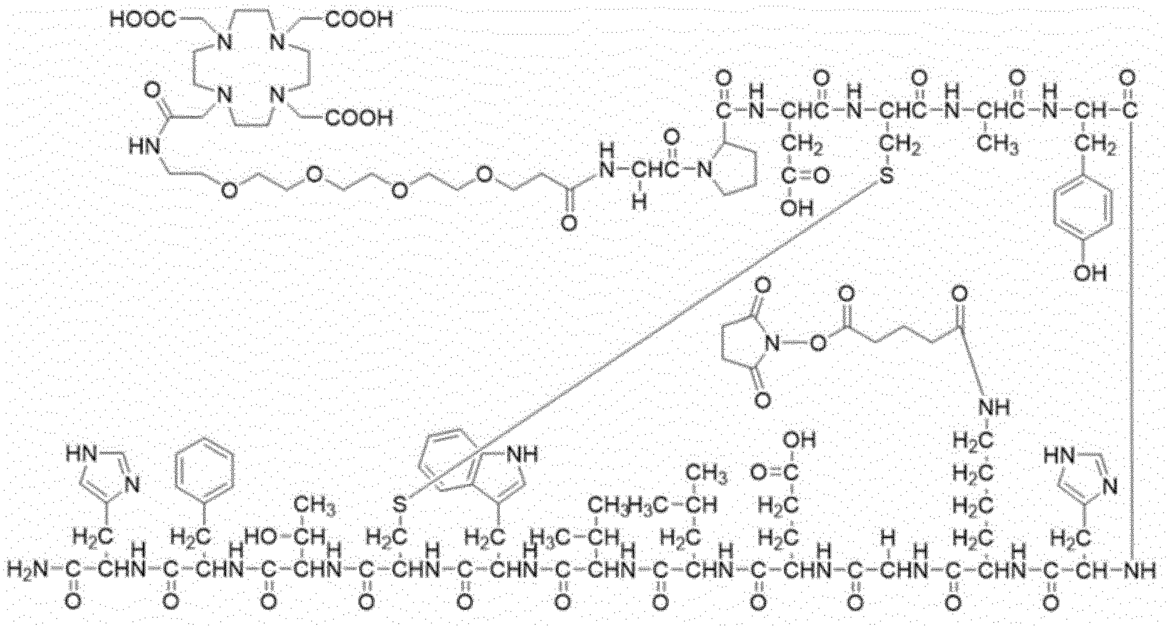

The above-mentioned labeling precursor had a structure in which DOTA as a chelate site is linked to the N-terminal of an antibody-modification peptide (peptide containing 17 amino acid residues having the same sequence as the sequence of SEQ ID NO: 10 in which Xaa2 is a lysine residue) via 8 polyethylene glycols, and linked to the 252nd lysine residue by EU numbering in various antibodies via an N-hydroxysuccinimidoester group in the structure.

This labeled precursor (450 μg) was dissolved in 4-(2-hydroxyethyl)-1-piperazine ethanesulfonic acid buffer (pH 5.5) as a solvent at 100 mmol/L. This solution was mixed with a $^{111}In$ ion-containing solution (indium ($^{111}In$) chloride

Injection, manufactured by Nihon Medi-Physics Co., Ltd.) as a radioactive metal source at 10 MBq as a radioactive amount, and a labeling reaction was performed at 45°C for 120 min.

The radiochemical yield, radiochemical purity, and amount of radioactivity administered to animal of various $^{111}$In-labeled antibodies are shown in Table 5. The radiochemical yield here refers to the amount of radioactivity of the $^{111}$In-labeled antibody with respect to the amount of radioactivity of $^{111}$In used. For the radioactivity measurement, a radioisotope Doze Calibrator (manufactured by CAPINTEC, model number: CRC-15R) was used. The radiochemical purity refers to the ratio (%) of the peak radioactivity count corresponding to the $^{111}$In-labeled antibody to the total radioactivity count of the filter paper as analyzed by filter paper chromatography. For filter paper chromatography, filter: manufactured by ADVANTEC, model number: No. 590, developing solvent: 0.01% EDTA, 50 mM citric acid-sodium citrate aqueous solution) was developed, and the radioactivity count was detected using a radio γ-TLC analyzer (manufactured by raytest, MODEL GITA Star).

[Table 5]

| $^{111}$In labeling information | | | | | |
|---|---|---|---|---|---|
| name of antibody | chimeric antibody | humanized antibody | | | |
| | | H01L03 | H01L04 | H02L04 | H04L04 |
| radiochemical yield (%) | 99.0 | 96.3 | 93.6 | 91.0 | 94.0 |
| radiochemical purity (%) | 100 | | | | |
| administered radioactivity amount (MBq) | 5.3±1.0 | 5.5±0.1 | 4.710.2 | 4.6±0.2 | 4.6±1.3 |
| (mean±standard deviation, n=4) | | | | | |

Reference Example 1-2: Biodistribution using tumor-bearing mice

(Production method of tumor-bearing mouse)

Human pancreatic cancer cell line SW1990 (0.7×10$^7$ cells) were subcutaneously administered to Balb/c nude mice (male) from the flank to the back thereof.

When the tumor size reached about 150-300 mm$^3$ 14 days after transplantation of SW1990, various $^{111}$In-labeled antibodies prepared in Reference Example 1-1 were administered from the tail vein of the mice. The tumor volume was calculated from the following calculation formula.

$$\text{tumor volume} = (\text{tumor minor axis}^2 \times \text{tumor major axis})/2$$

[Table 6]

| Animal information (on administration of each $^{111}$In-labeled antibody) | | | | | |
|---|---|---|---|---|---|
| name of antibody | chimeric antibody | humanized antibody | | | |
| | | H01L03 | H01L04 | H02L04 | H04L04 |
| tumor volume (mm$^3$) | 203±110 | 201145 | 285±24 | 209183 | 193±80 |
| body weight (g) | 23.211.3 | 23.410.6 | 23.5±0.7 | 22.111.7 | 21.211.8 |
| (mean±standard deviation, n=4) | | | | | |

(Evaluation method)

SPECT-CT imaging (small animal SPECT-CT apparatus: FX-3000, manufactured by Trifoil) was performed under the conditions in the following Table. The time points for imaging were 1, 6, 24, 48, 72, and 168 hr after administration. Image reconstruction was performed by the OSEM method for SPECT and the FBP method for CT. A VOI (volume of interest, three-dimensional ROI) analysis of the tumor and liver at each time point was performed. The count number per organ volume was corrected to %ID/g, the physical half-life was converted from $^{111}$In to $^{225}$Ac, the difference in physical half-life was corrected, and the time activity curve with the biological half-life added was obtained. The cumulative

radioactivity amount was calculated from the integrated value of this time activity curve, and the absorbed dose was calculated in a sphere model (OLINDA/EXM ver2.0), and this was compared and examined for each antibody. However, for H01L03, a decrease in the time activity curve was not observed 168 hr after administration. Thus, the biological half-life was not considered and only the physical half-life was added.

[Table 7]

| SPECT imaging conditions | |
|---|---|
| Isotope | Indium-111 medium +high energy |
| Collimator | MMP952 |
| Radius of Rotation | 50 mm |
| Projection Limit | 150 sec |
| Projection Count | 16 |
| Rotation Angle | 90 degree |

[Table 8]

| CT imaging conditions | |
|---|---|
| Projection count | 207 views |
| Frames averaged | 1 frames |
| Detector binding | 2 x 2 |
| X-ray tube current | 270 uA |
| X-ray tube voltage | 60 kV |
| Exposure time | 230 ms |
| Magnification | 2.0 |

(Results)

The results of performing SPECT-CT imaging are shown in Fig. 9. The results of VOI analysis of the tumor and liver at each time point are shown in Fig. 10. A graph showing the results of confirming the biodistribution and the excretion route after the completion of SPECT-CT imaging 168 hr after administration are shown in Fig. 11.

The accumulation in the tumor was highest when the humanized antibody (H01L03) was administered, and lowest when the chimeric antibody was administered. The accumulation in the liver was highest when chimeric antibody was administered, and lower when any humanized antibody was administered as compared to the chimeric antibody. When the humanized antibody (H01L03) was administered, the excretion was the slowest and the blood retention was high. Regardless of which antibody was administered, the accumulation in the liver and spleen was high among the normal organs, followed by the accumulation in the lung and kidney.

When the threshold dose of liver was assumed to be 30 Gy, the maximum tolerated dose was estimated to be 3.90 MBq with a humanized antibody (H01L03) and 0.43 MBq with a chimeric antibody. The maximum tolerated dose (MBq) was calculated by threshold dose (Gy)/absorbed dose (Gy/MBq).

Reference Example 1-3: in vitro autoradiography

The results of images evaluating the binding property and specificity of various antibodies for MUC5AC by in vitro autoradiography (ARG) using various $^{111}$In-labeled antibodies prepared in Reference Example 1-1 are shown in Fig. 12. In addition, a graph of the numerical values of the radioactivity density (Bq/mm$^2$) in the ROI calculated by setting the region of interest (ROI) in the entire section is shown in Fig. 13.

A section with a thickness of 10 um was prepared using a cryostat (manufactured by Leica) from a frozen block obtained by freezing MUC5AC high expression tumor (SW1990 transplanted tumor tissue) or MUC5AC low expression tumor (MIAPaCa2 transplanted tumor tissue) in liquid nitrogen and used for in vitro ARG.

A section stored at -80°C was returned to room temperature, dried for 30 min or longer, immersed in phosphate

buffered saline for 30 min, and then in phosphate buffered saline containing 1 % by volume bovine serum albumin for 30 min, whereby the section was hydrophilized.

The hydrophilized cryosection was immersed in 1% by volume bovine serum albumin-containing phosphate buffered saline containing 5 kBq/mL various $^{111}$In-labeled antibodies for 30 min each. Then, the section was washed by immersing for 5 min in each of 1% by volume bovine serum albumin-containing phosphate buffered saline, phosphate buffered saline, and phosphate buffered saline in this order. The section after washing was air dried, exposed in an imaging plate (BAS-SR2040, manufactured by FUJIFILM Wako Pure Chemical Corporation) for about 15 hr, and an autoradiogram was obtained using a fluoroimage analyzer (Typhoon FLA 7000 IP, manufactured by GE Healthcare). Using the analysis software "Image Quant TL" attached to the fluoroimage analyzer, the obtained autoradiogram was used to set the ROI over the entire section, and the radioactivity density (Bq/mm$^2$) in the ROI was calculated.

It was confirmed that all $^{111}$In-labeled humanized antibodies retained binding property and specificity for MUC5AC (Fig. 13, data of MUC5AC high expression tumor). It was confirmed that various humanized antibodies had less nonspecific binding as compared with the chimeric antibody (Fig. 13, data of MUC5AC low, non-expression tumor).

From the results of Reference Examples 1-2, 1-3, the humanized antibody has higher specificity for MUC5AC than the chimeric antibody, and has high accumulation in tumor and low accumulation in normal organs such as liver and the like. It was clarified that it provides a more superior delivery technique relating to RI-labeled antibodies.

The results of this Reference Example are summarized in the following Table. In the Table, the tumor volume was calculated assuming 150 mm$^3$ in the absorbed dose of SPECT. The absorbed dose of the liver was calculated based on the mean (1.15±0.14 g, n=19) of the weight of the mouse liver used in this Reference Example. The numerical value of the biodistribution was expressed by the mean±standard deviation of n=4 (however, n=3 for H02L04).

[Table 9]

| | | name of antibody | | | | |
|---|---|---|---|---|---|---|
| | | chimeric antibody | humanized antibody | | | |
| | | | H01L03 | H01L04 | H02L04 | H04L04 |
| SPECT absorbed dose | tumor (Gy/MBq) | 9.5 | 35.0 | 15.3 | 13.7 | 15.0 |
| | liver (Gy/MBq) | 70.1 | 7.7 | 33.4 | 27.0 | 35.4 |
| biodistribution at 168 hr after administration | tumor (%ID/g) | 11.6±0.9 | 24.213.9 | 12.912.6 | 15.6±6.2 | 17.214.2 |
| | liver (%ID/g) | 7.9±1.9 | 3.611.7 | 3.110.9 | 2.710.7 | 3.9±1.4 |
| | spleen (%ID/g) | 5.0±1.8 | 5.410.8 | 3.811.2 | 4.013.0 | 3.410.3 |
| | kidney (%ID/g) | 1.810.3 | 2.510.4 | 1.110.3 | 1.4±1.0 | 1.6±0.2 |
| | blood (%ID/g) | 2.210.6 | 5.311.3 | 2.110.8 | 2.612.3 | 3.810.7 |

While the present invention has been described above with reference to the embodiments, the present invention is not limited to the above-mentioned embodiments. Various changes that can be understood by those skilled in the art can be made to the constitution and details of the present invention within the scope of the present invention.

The contents disclosed in any publication cited herein, including patents and patent applications, are hereby incorporated in their entireties by reference, to the extent that they have been disclosed herein.

[Industrial Applicability]

According to the radioactive antitumor agent of the present invention, since a conjugate containing a humanized antibody that specifically binds to MUC5AC and Actinium-225 that emits α-rays is contained as an active ingredient, when used for RI internal therapy of cancer, it accumulates specifically in tumor expressing MUC5AC and can irradiate α-rays specifically on tumor cells without affecting normal cells, whereby higher safety and higher therapeutic effect is obtained. Furthermore, since it is used in combination with a drug for reducing renal toxicity, side effects feared in cancer therapy are reduced.

This application is based on a patent application No. 2021-072206 filed in Japan (filing date: April 21, 2021), the contents of which are incorporated in full herein.

## Claims

1. A radioactive antitumor agent comprising a $^{225}$Ac-labeled anti-MUC5AC humanized antibody (humanized antibody labeled with actinium-225 that specifically binds to mucin subtype 5AC) as an active ingredient, which is used in combination with a drug for reducing renal toxicity.

2. A radioactive antitumor agent comprising a $^{225}$Ac-labeled anti-MUC5AC humanized antibody (humanized antibody labeled with actinium-225 that specifically binds to mucin subtype 5AC) and a drug for reducing renal toxicity as active ingredients.

3. The radioactive antitumor agent according to claim 1 or 2, wherein the drug for reducing renal toxicity is a drug for reducing renal toxicity caused by the administration of a drug containing Actinium-225.

4. The radioactive antitumor agent according to claim 1 or 2, wherein the drug for reducing renal toxicity is a drug for reducing renal toxicity caused by Actinium-225 or a daughter nuclide of Actinium-225.

5. The radioactive antitumor agent according to claim 1 or 2, wherein the aforementioned renal toxicity is late renal toxicity that occurs after a lapse of not less than 10 weeks after administration of the aforementioned radioactive antitumor agent.

6. The radioactive antitumor agent according to claim 1 or 2, wherein the aforementioned drug for reducing renal toxicity is a diuretic or a metal scavenger.

7. The radioactive antitumor agent according to claim 6, wherein the aforementioned metal scavenger comprises DTPA, Ca-DTPA, or Zn-DTPA.

8. The radioactive antitumor agent according to claim 6, wherein the aforementioned diuretic comprises furosemide, spironolactone, or eplerenone.

9. The radioactive antitumor agent according to claim 1 or 2, wherein the aforementioned anti-MUC5AC humanized antibody comprises

a heavy chain variable region consisting of

(1) the amino acid sequence shown in SEQ ID NO: 1 (H01), or an amino acid sequence having not less than 95% sequence identity with the amino acid sequence shown in SEQ ID NO: 1;
(2) the amino acid sequence shown in SEQ ID NO: 2 (H02), or an amino acid sequence having not less than 95% sequence identity with the amino acid sequence shown in SEQ ID NO: 2;
(3) the amino acid sequence shown in SEQ ID NO: 3 (H03), or an amino acid sequence having not less than 95% sequence identity with the amino acid sequence shown in SEQ ID NO: 3; or
(4) the amino acid sequence shown in SEQ ID NO: 4 (H04), or an amino acid sequence having not less than 95% sequence identity with the amino acid sequence shown in SEQ ID NO: 4; and

a light chain variable region consisting of

(5) the amino acid sequence shown in SEQ ID NO: 5 (L01), or an amino acid sequence having not less than 95% sequence identity with the amino acid sequence shown in SEQ ID NO: 5;
(6) the amino acid sequence shown in SEQ ID NO: 6 (L02), or an amino acid sequence having not less than 95% sequence identity with the amino acid sequence shown in SEQ ID NO: 6;
(7) the amino acid sequence shown in SEQ ID NO: 7 (L03), or an amino acid sequence having not less than 95% sequence identity with the amino acid sequence shown in SEQ ID NO: 7; or
(8) the amino acid sequence shown in SEQ ID NO: 8 (L04), or an amino acid sequence having not less than 95% sequence identity with the amino acid sequence shown in SEQ ID NO: 8.

10. The radioactive antitumor agent according to claim 1 or 2, wherein the aforementioned anti-MUC5AC humanized antibody is a humanized antibody having

(1) a heavy chain variable region consisting of the amino acid sequence shown by SEQ ID NO: 1 (H01) or an

amino acid sequence having 95% or more sequence identity with the amino acid sequence shown by SEQ ID NO: 1, and

(7) a light chain variable region consisting of the amino acid sequence shown by SEQ ID NO: 7 (L03) or an amino acid sequence having 95% or more sequence identity with the amino acid sequence shown by SEQ ID NO: 7.

**11.** The radioactive antitumor agent according to claim 1 or 2, wherein the aforementioned $^{225}Ac$-labeled anti-MUC5AC humanized antibody is a conjugate of a chelating agent complexed with actinium-225 and the aforementioned anti-MUC5AC humanized antibody, and the Fc region of the aforementioned anti-MUC5AC humanized antibody is site-specifically modified with the aforementioned chelating agent via a linker.

**12.** The radioactive antitumor agent according to claim 11, wherein the aforementioned linker comprises a peptide represented by the following formula (i) which consists of not less than 13 and not more than 17 amino acid residues, and which is formed by a crosslinking reaction of the aforementioned peptide modified by a crosslinking agent and the aforementioned antibody:

(Xa)-Xaa1-(Xb)-Xaa2-(Xc)-Xaa3-(Xd)··· (i)

wherein Xa, Xb, Xc and Xd are continuous X in the number of a, continuous X in the number of b, continuous X in the number of c, and continuous X in the number of d, respectively,

X is an amino acid residue having neither a thiol group nor a haloacetyl group in the side chain,

a, b, c and d are each independently an integer of not less than one and not more than 5, and satisfy $a+b+c+d \leq 14$,

Xaa1 and Xaa3 are each independently an amino acid residue derived from an amino acid having a thiol group in the side chain, or

one is an amino acid residue derived from an amino acid having a thiol group in the side chain and the other is an amino acid residue derived from an amino acid having a haloacetyl group in the side chain, and Xaa1 and Xaa3 are linked, and

Xaa2 is a lysine residue, an arginine residue, a cysteine residue, an aspartic acid residue, a glutamic acid residue, 2-aminosuberic acid, or diamino propionic acid, and modified with the aforementioned crosslinking agent.

**13.** The radioactive antitumor agent according to claim 11, wherein the aforementioned chelating agent has a structure derived from a compound represented by the following formula (A) or a salt thereof:

(A)

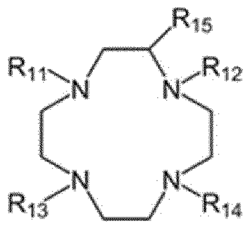

wherein in the formula (A), $R_{11}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the antibody, p is an integer of not less than 0 and not more than 3, $R_{15}$ is a hydrogen atom when $R_{12}$ is a substituent for conjugating with the antibody, and $R_{15}$ is a substituent for conjugating with the antibody when $R_{12}$ is not a substituent for conjugating with the antibody.

**14.** The radioactive antitumor agent according to claim 1 or 2, which is used to treat pancreatic cancer, thyroid cancer, liver cancer, colorectal cancer, stomach cancer, urothelial cancer, breast cancer, cervical cancer, ovarian cancer, endometrial cancer or bile duct cancer.

**15.** A drug for reducing renal toxicity, which is used in combination with a radioactive antitumor agent comprising $^{225}Ac$-labeled anti-MUC5AC humanized antibody as an active ingredient.

**16.** The drug according to claim 15, wherein the drug for reducing renal toxicity is a diuretic or a metal scavenger.

**17.** A combination drug comprising a radioactive antitumor agent comprising a $^{225}$Ac-labeled anti-MUC5AC humanized antibody as an active ingredient, and a drug for reducing renal toxicity.

[Fig. 1]

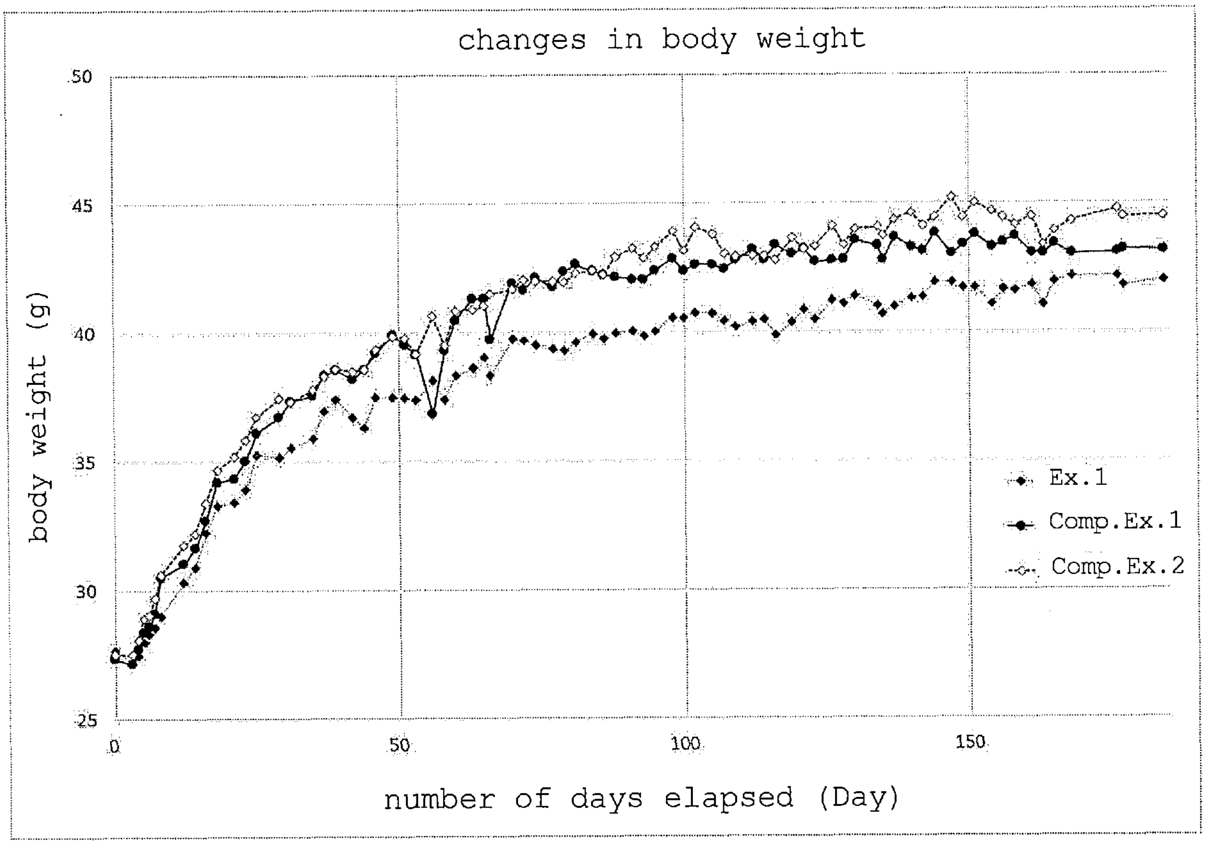
changes in body weight
body weight (g)
50
45
40
35
30
25
0
50
100
150
number of days elapsed (Day)
Ex.1
Comp.Ex.1
Comp.Ex.2

[Fig. 2]

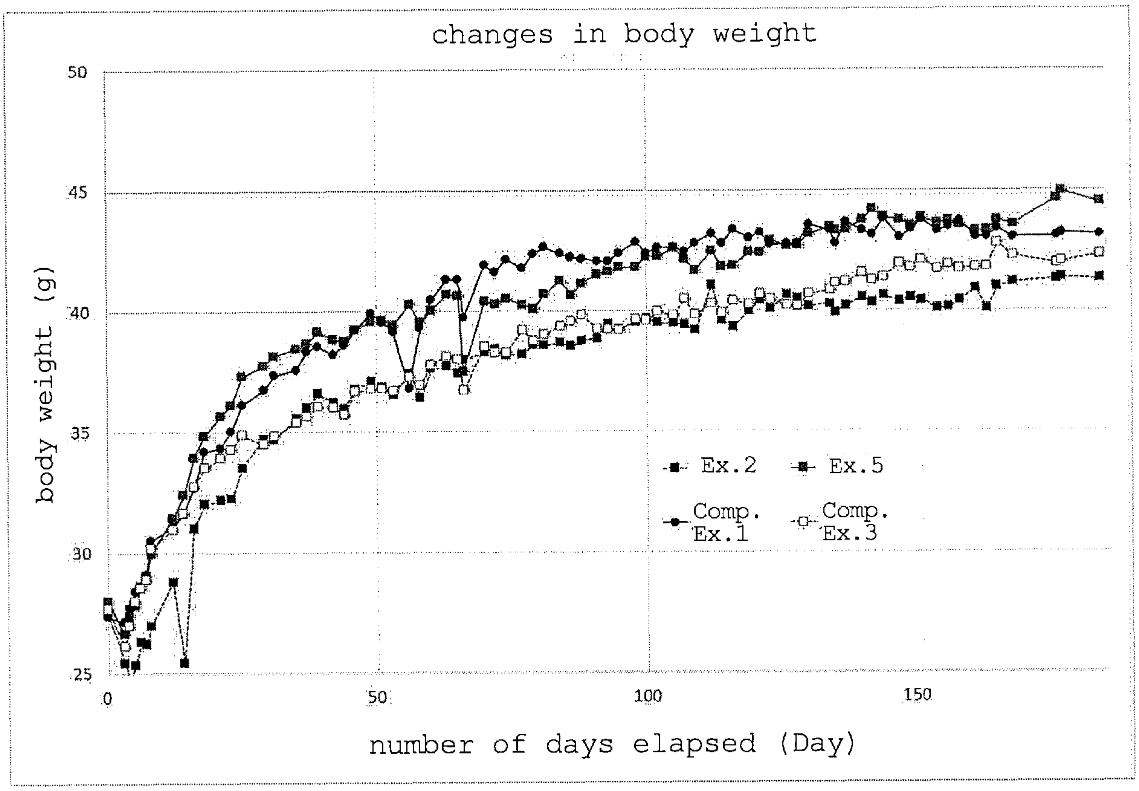
changes in body weight
50
45
40
35
30
25
body weight (g)
0
50
100
150
number of days elapsed (Day)
Ex.2
Ex.5
Comp. Ex.1
Comp. Ex.3

[Fig. 3]

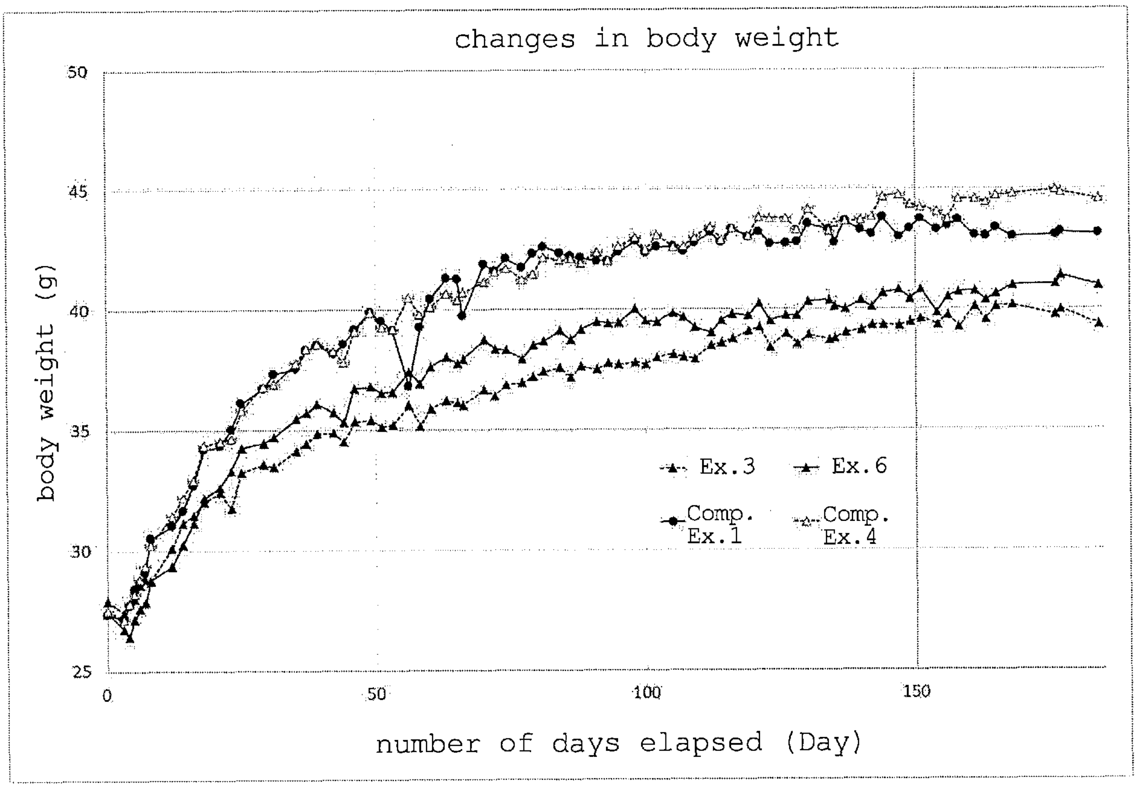
changes in body weight
50
45
40
35
30
25
body weight (g)
0
50
100
150
number of days elapsed (Day)
Ex.3
Ex.6
Comp. Ex.1
Comp. Ex.4

[Fig. 4]

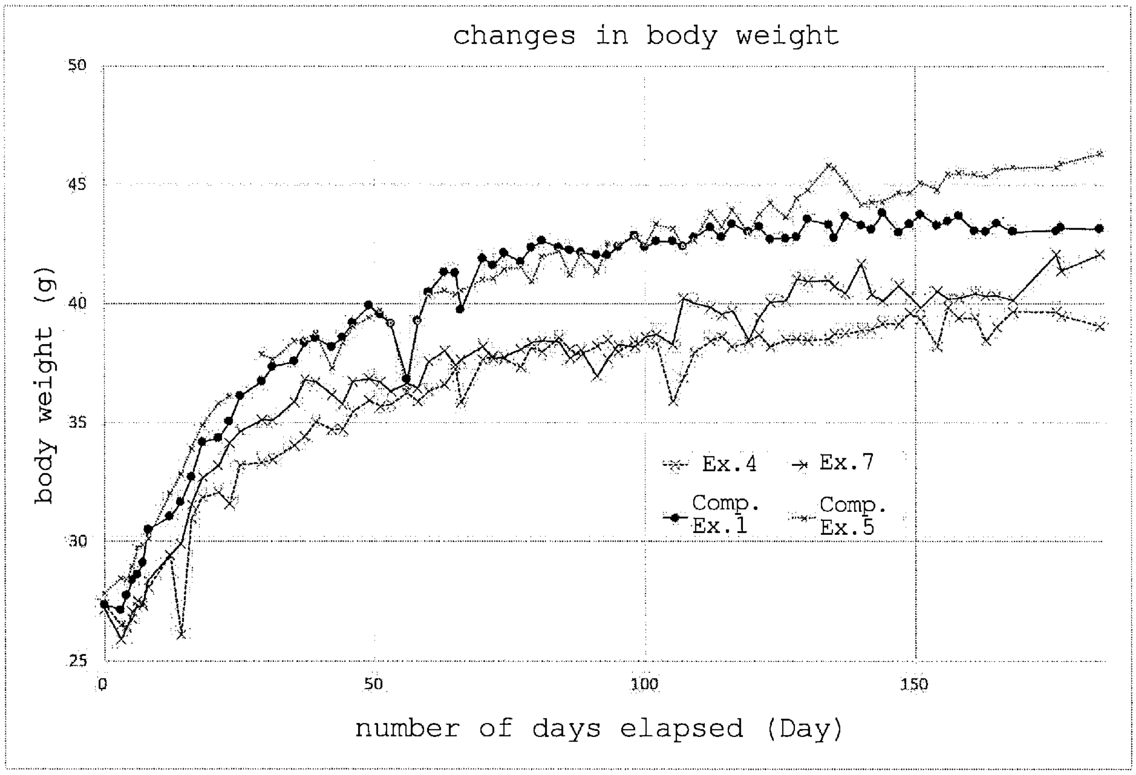
changes in body weight
body weight (g)
50
45
40
35
30
25
0
50
100
150
number of days elapsed (Day)
Ex.4
Ex.7
Comp. Ex.1
Comp. Ex.5

[Fig. 5]

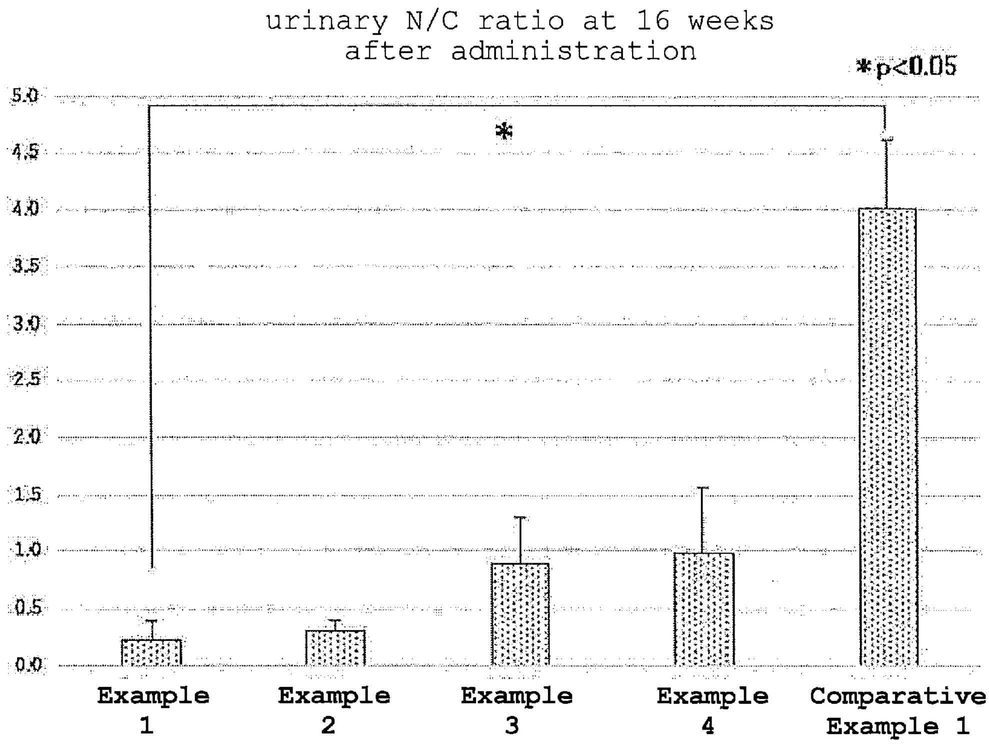
urinary N/C ratio at 16 weeks after administration
*p<0.05
*
5.0
4.5
4.0
3.5
3.0
2.5
2.0
1.5
1.0
0.5
0.0
Example 1
Example 2
Example 3
Example 4
Comparative Example 1

[Fig. 6]

| findings | | Example 1 | Example 2 | Example 3 | Example 4 | Example 5 | Example 6 | Example 7 | Comparative Example 1 |
|---|---|---|---|---|---|---|---|---|---|
| | number of individuals | 5 | 5 | 5 | 5 | 4 | 5 | 2 | 5 |
| kidney | grade | | | | | | | | |
| degeneration/regeneration of renal tubular epithelium in outer layer of medulla | - | 1 | 1 | 1 | 1 | 0 | 0 | 1 | 0 |
| | + | 3 | 3 | 2 | 2 | 2 | 4 | 1 | 0 |
| | 2+ | 1 | 1 | 2 | 1 | 2 | 1 | 0 | 5 |
| | 3+ | 0 | 0 | 0 | 1 | 0 | 0 | 0 | 0 |
| basophilic tubules with inflammatory cell infiltration and fibrosis | - | 1 | 0 | 0 | 0 | 0 | 0 | 0 | 0 |
| | + | 3 | 5 | 2 | 2 | 4 | 3 | 2 | 3 |
| | 2+ | 1 | 0 | 3 | 2 | 0 | 0 | 0 | 2 |
| | 3+ | 0 | 0 | 0 | 1 | 0 | 2 | 0 | 0 |

[Fig. 7]

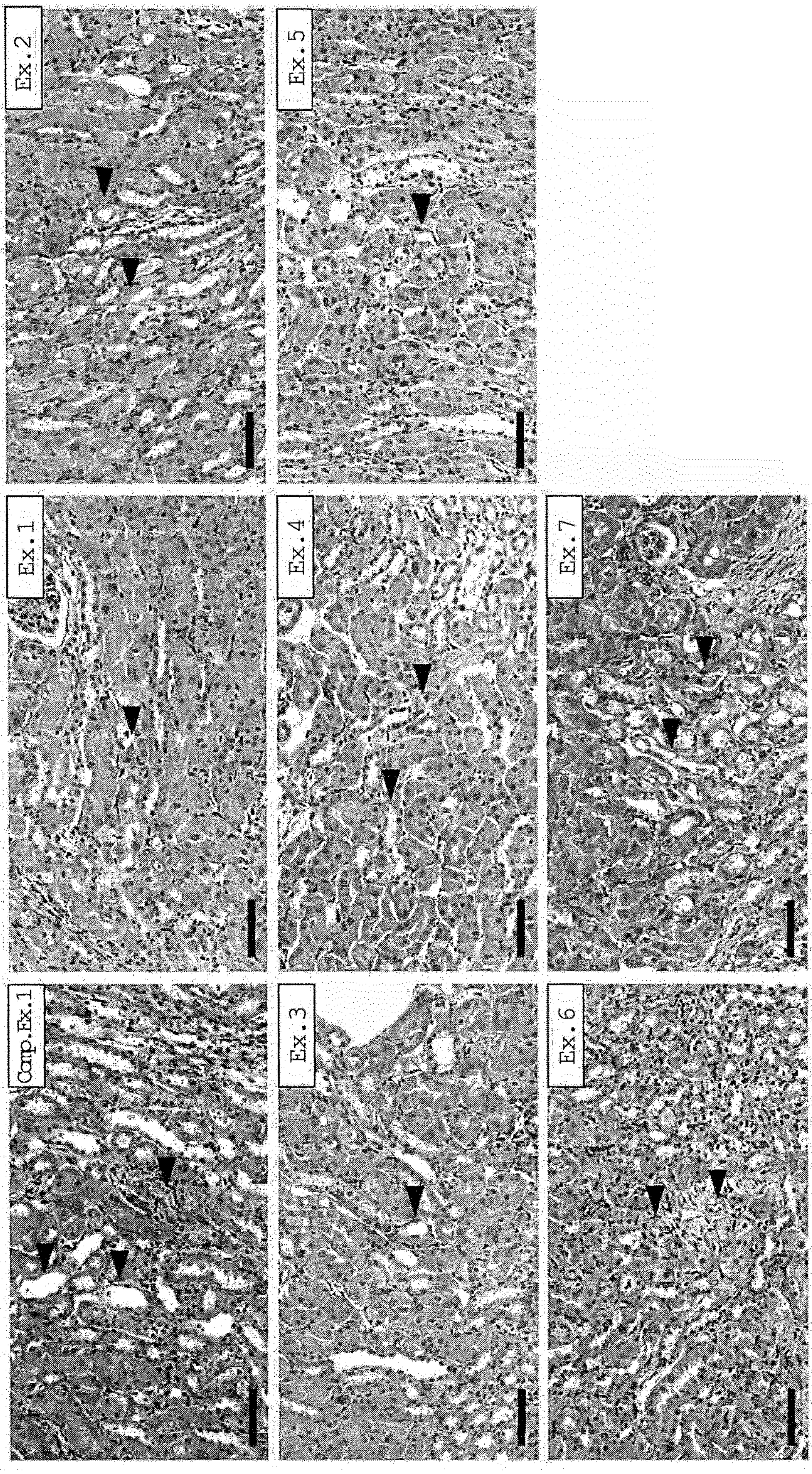

[Fig. 8]

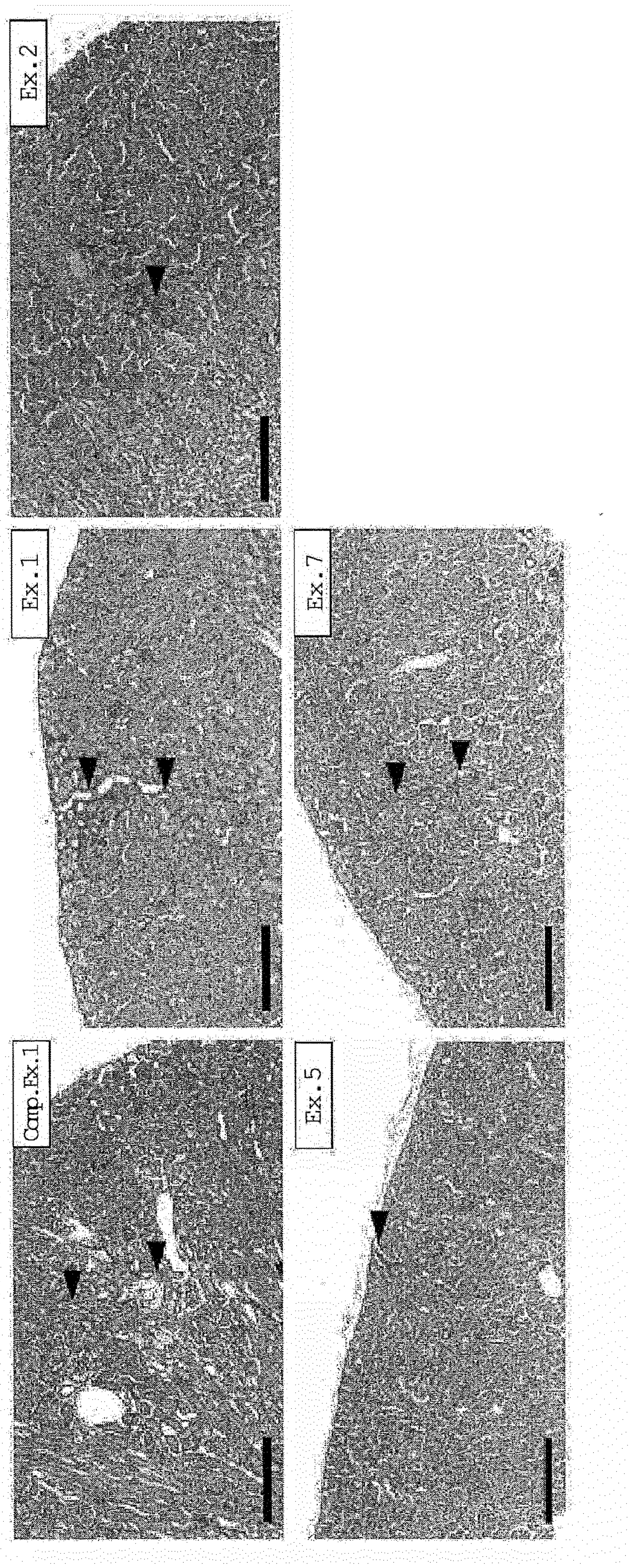

[Fig. 9]

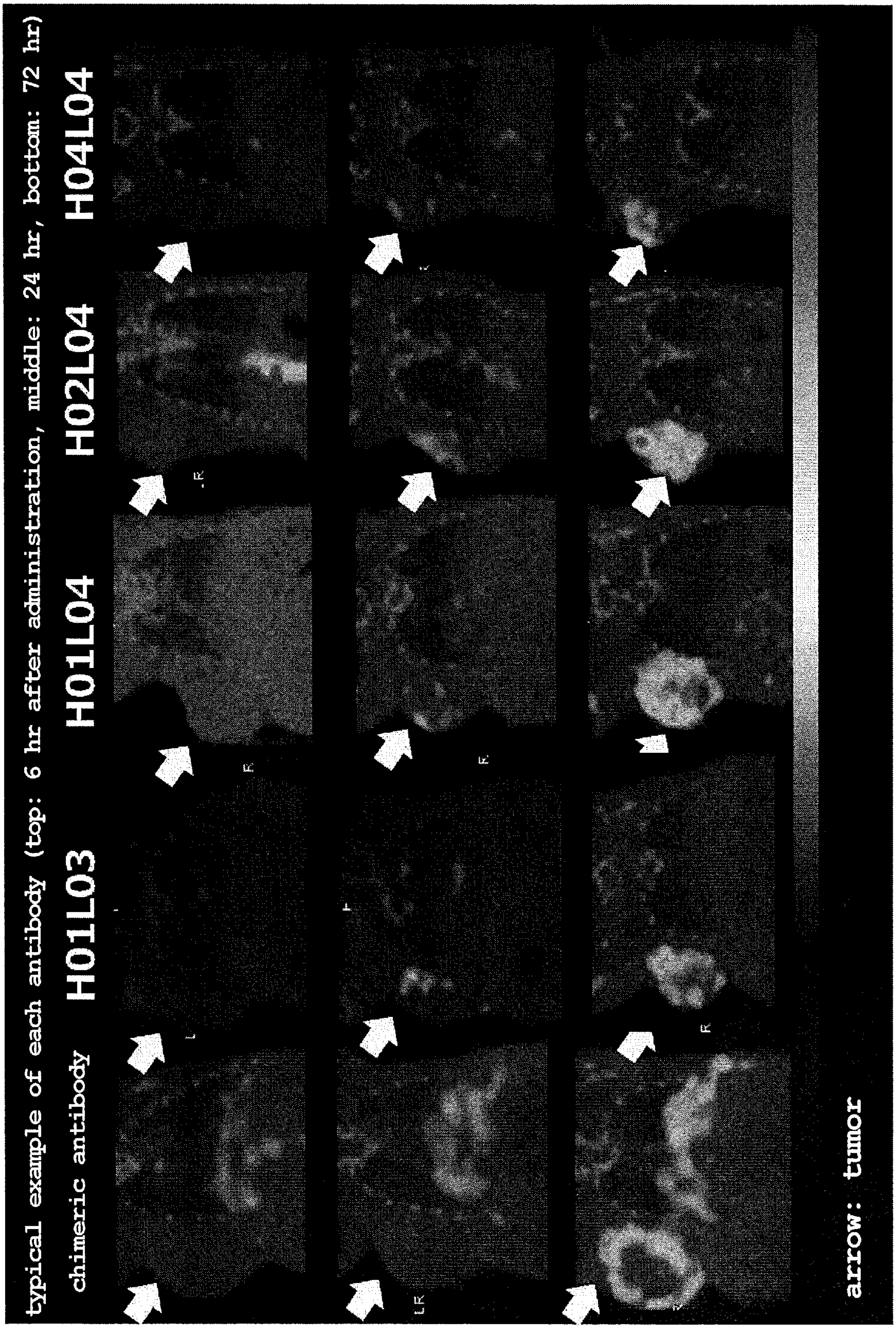
typical example of each antibody (top: 6 hr after administration, middle: 24 hr, bottom: 72 hr)
chimeric antibody
H01L03
H01L04
H02L04
H04L04
arrow: tumor

[Fig. 10]

(tumor)

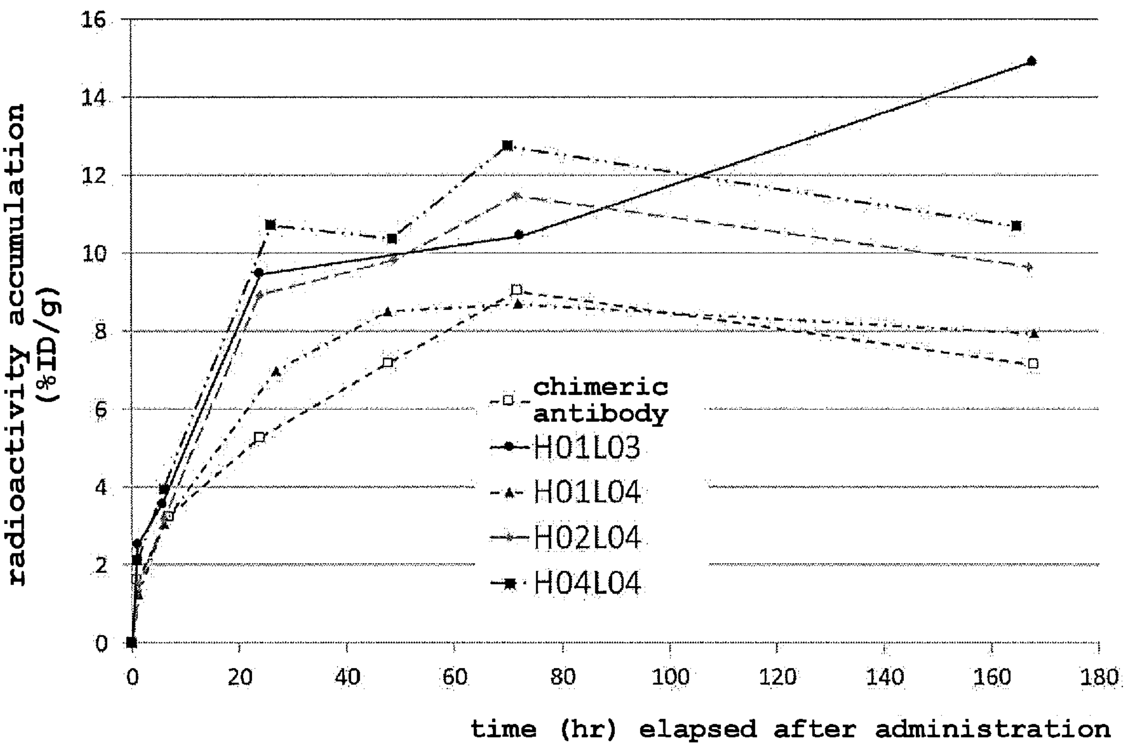


(liver)

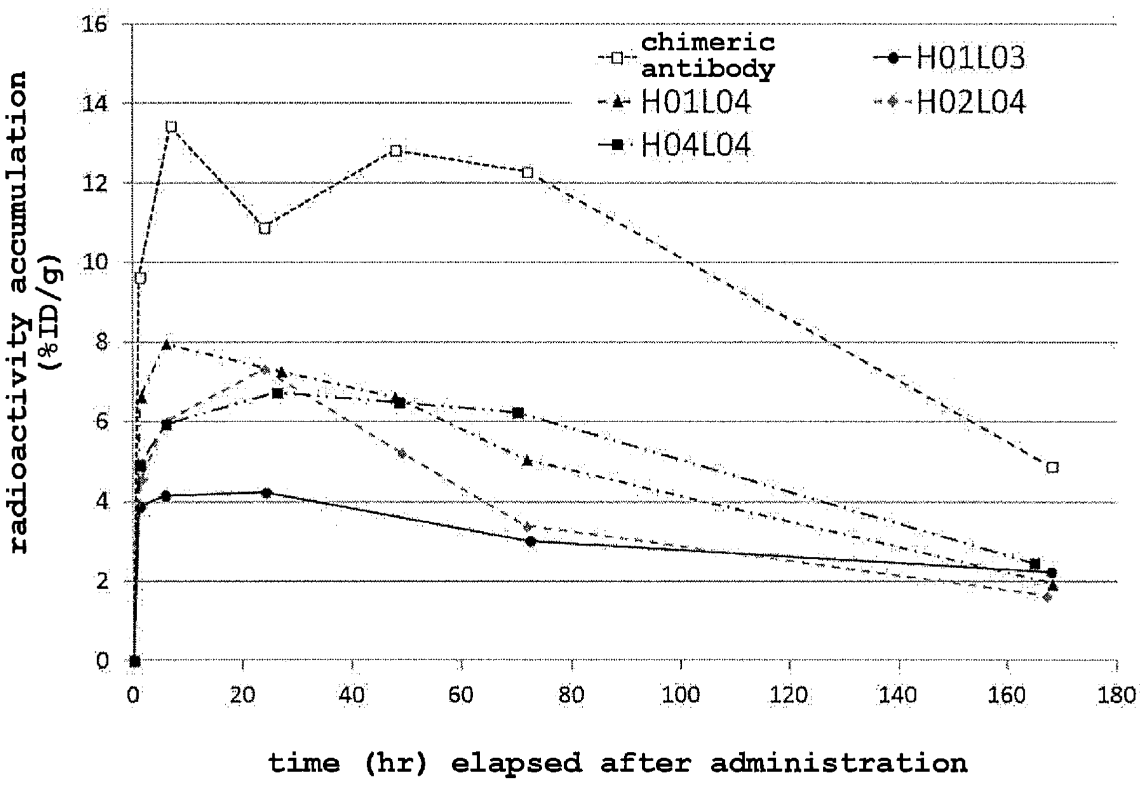

[Fig. 11]

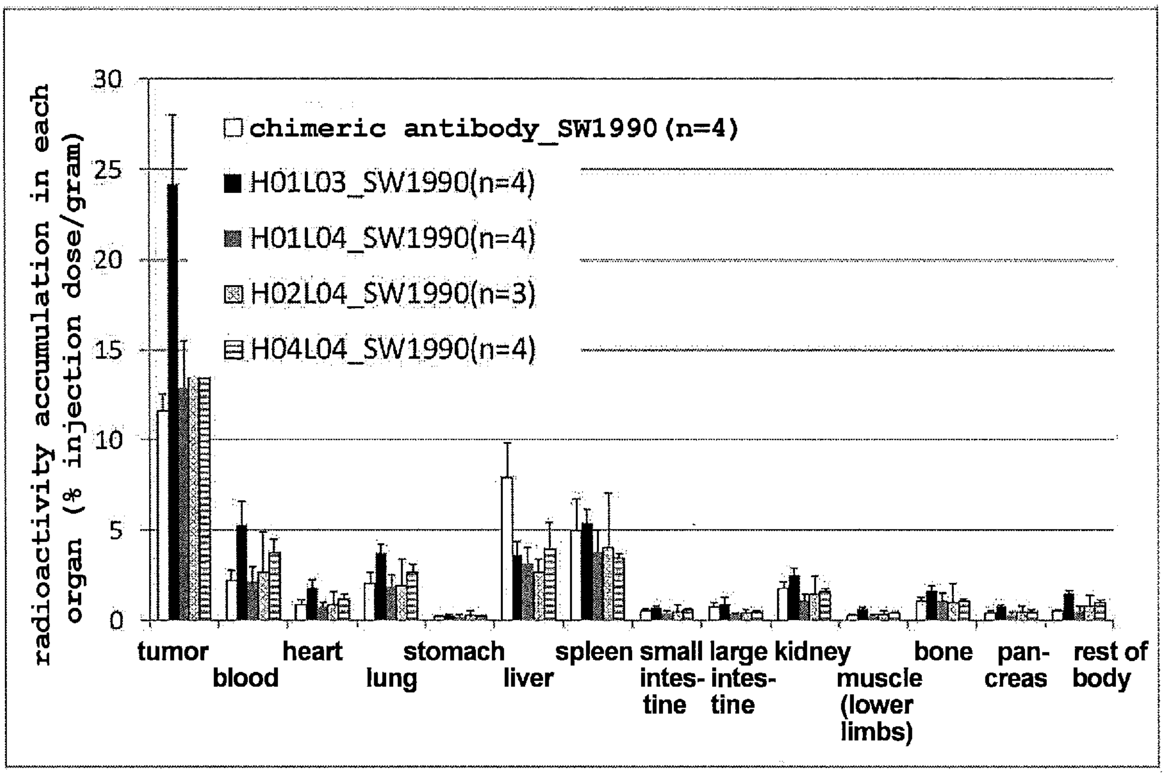
radioactivity accumulation in each organ (% injection dose/gram)
30
25
20
15
10
5
0
chimeric antibody_SW1990(n=4)
H01L03_SW1990(n=4)
H01L04_SW1990(n=4)
H02L04_SW1990(n=3)
H04L04_SW1990(n=4)
tumor
blood
heart
lung
stomach
liver
spleen
small intes- tine
large intes- tine
kidney
muscle (lower limbs)
bone
pan- creas
rest of body

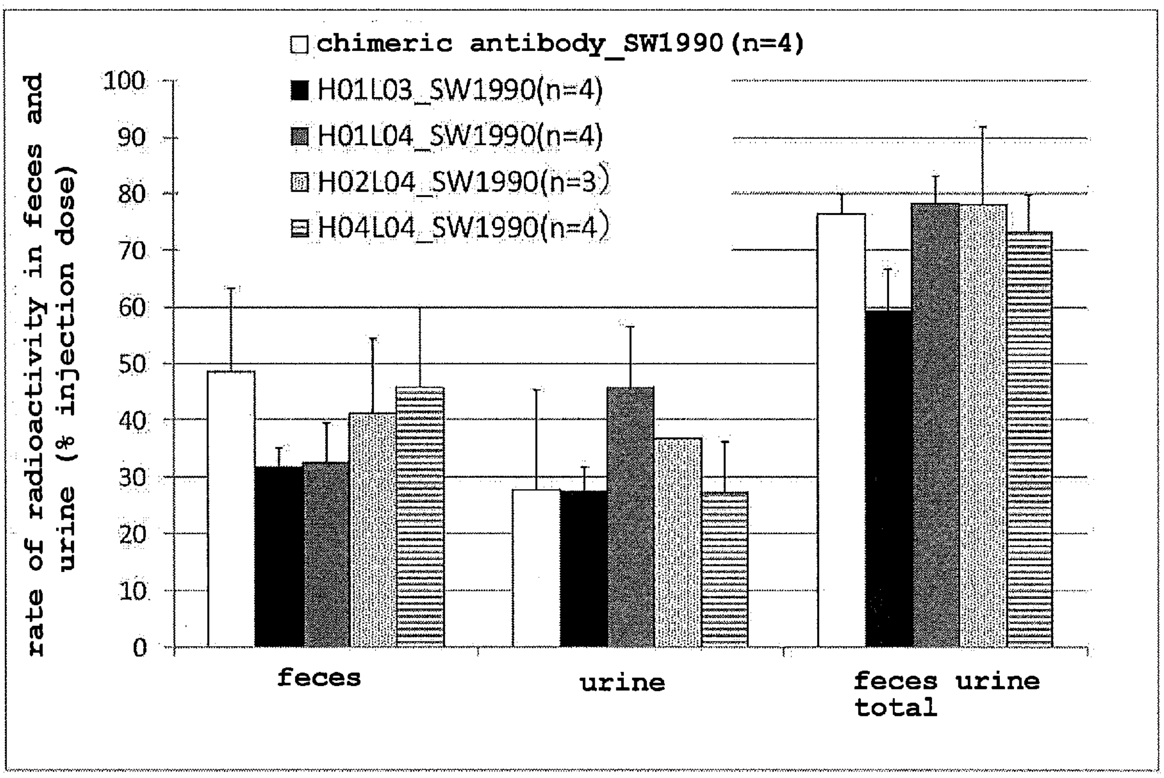
rate of radioactivity in feces and urine (% injection dose)
100
90
80
70
60
50
40
30
20
10
0
chimeric antibody_SW1990(n=4)
H01L03_SW1990(n=4)
H01L04_SW1990(n=4)
H02L04_SW1990(n=3)
H04L04_SW1990(n=4)
feces
urine
feces urine total

[Fig. 12]

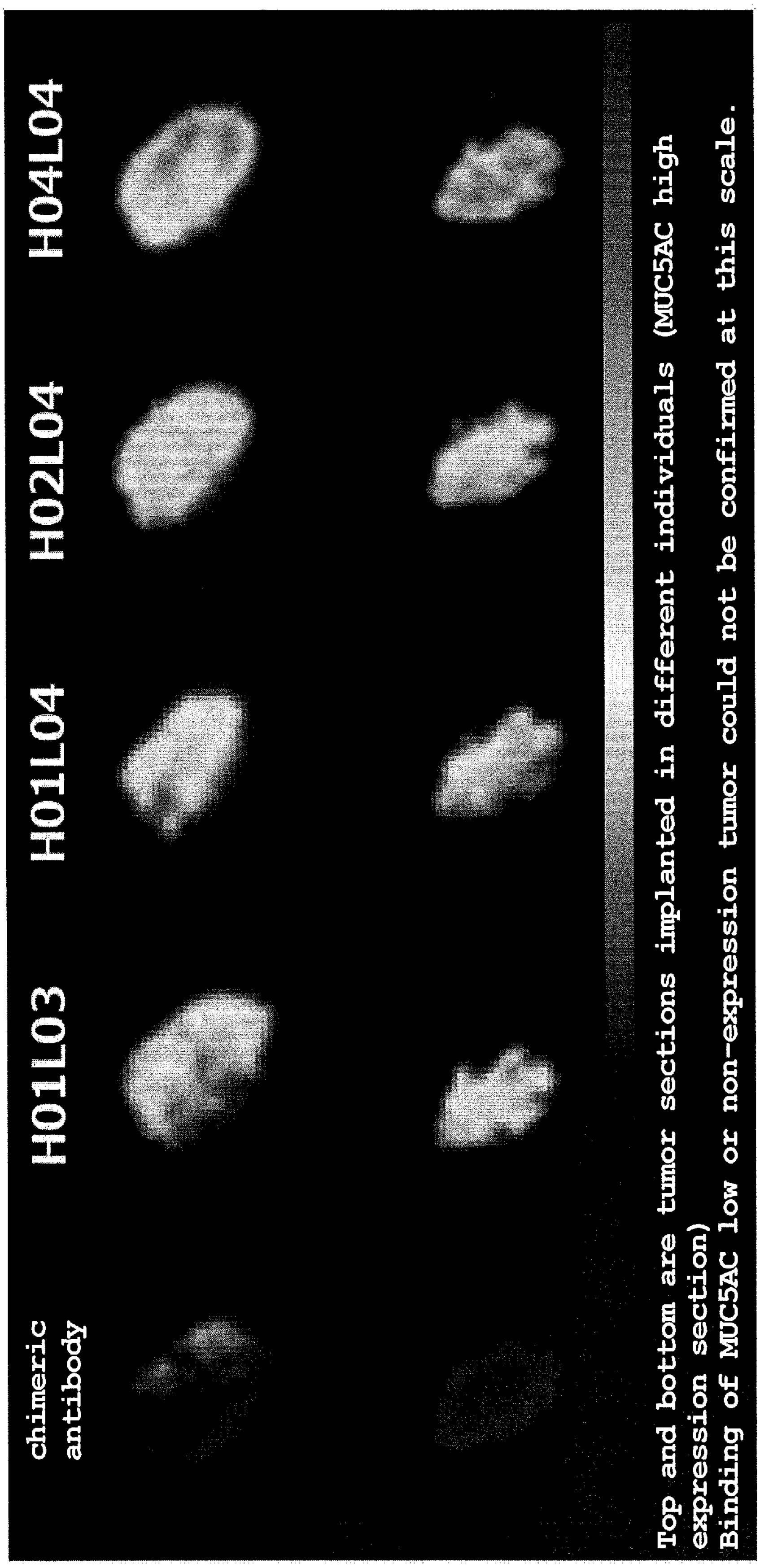
chimeric antibody
H01L03
H01L04
H02L04
H04L04
Top and bottom are tumor sections implanted in different individuals (MUC5AC high expression section)
Binding of MUC5AC low or non-expression tumor could not be confirmed at this scale.

[Fig. 13]

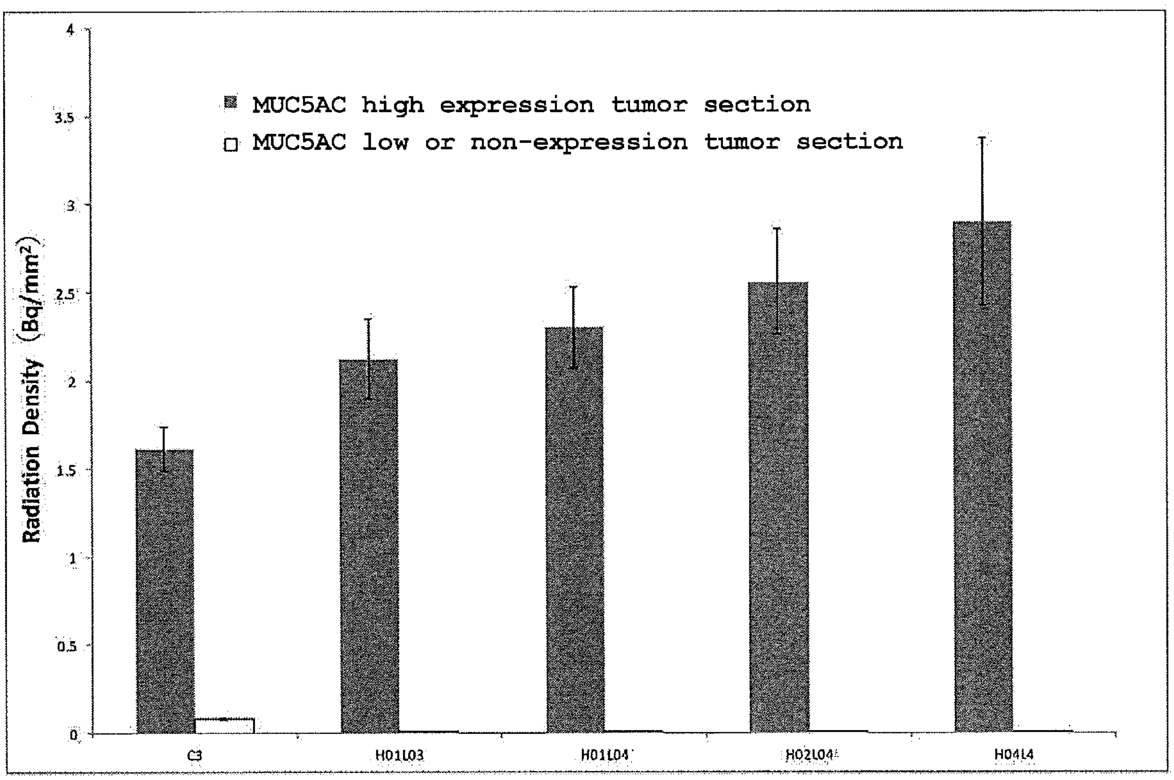
MUC5AC high expression tumor section
MUC5AC low or non-expression tumor section
Radiation Density (Bq/mm2)
4
3.5
3
2.5
2
1.5
1
0.5
0
C3
H01L03
H01L04
H02L04
H04L4

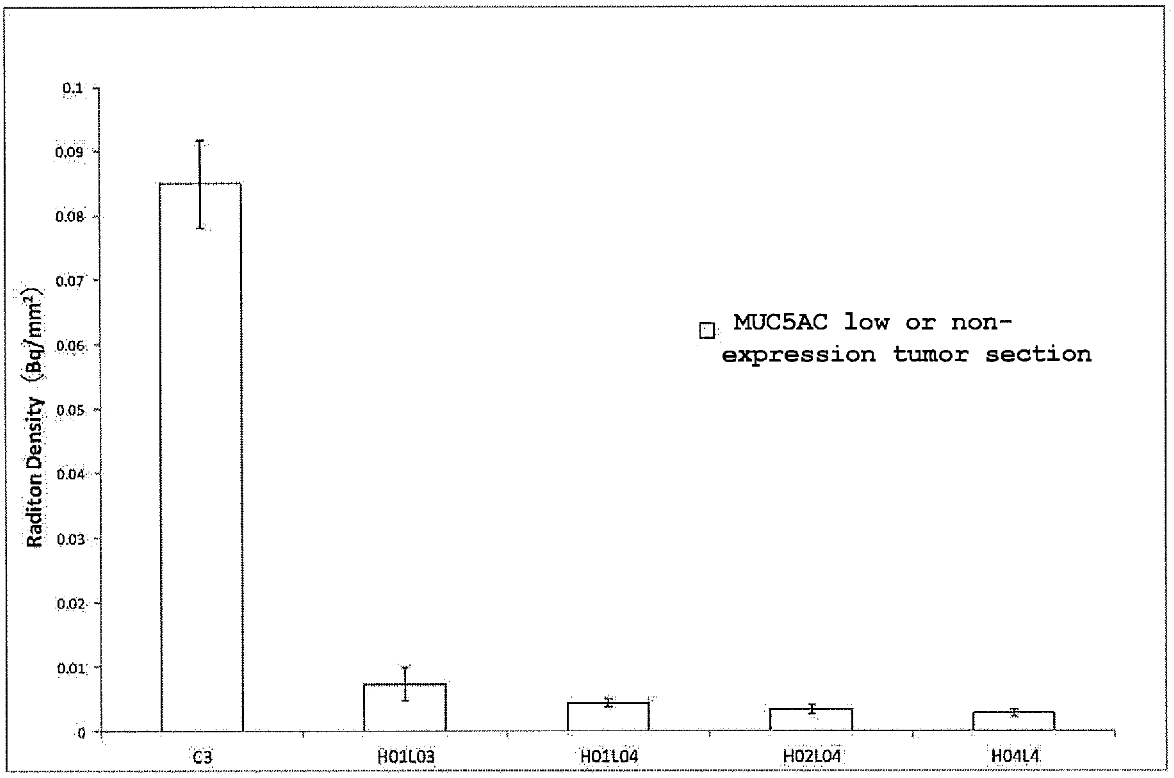
MUC5AC low or non-expression tumor section
Raditon Density (Bq/mm2)
0.1
0.09
0.08
0.07
0.06
0.05
0.04
0.03
0.02
0.01
0
C3
H01L03
H01L04
H02L04
H04L4

**INTERNATIONAL SEARCH REPORT**

International application No.
**PCT/JP2022/018414**

**A. CLASSIFICATION OF SUBJECT MATTER**

***A61K 51/10***(2006.01)i; ***A61K 31/198***(2006.01)i; ***A61K 31/341***(2006.01)i; ***A61K 31/585***(2006.01)i; ***A61K 33/24***(2019.01)i; ***A61K 39/395***(2006.01)i; ***A61K 45/00***(2006.01)i; ***A61K 47/22***(2006.01)i; ***A61K 47/69***(2017.01)i; ***A61P 13/12***(2006.01)i; ***A61P 35/00***(2006.01)i; ***A61P 39/04***(2006.01)i; ***A61P 43/00***(2006.01)i; ***C07K 16/18***(2006.01)i; ***C07K 16/46***(2006.01)i
FI: A61K51/10 100; A61K31/198; A61K31/341; A61K31/585; A61K33/24; A61K39/395 T; A61K39/395 Y; A61K45/00; A61K47/22; A61K47/69; A61P13/12; A61P35/00; A61P39/04; A61P43/00 111; C07K16/18 ZNA; C07K16/46

According to International Patent Classification (IPC) or to both national classification and IPC

**B. FIELDS SEARCHED**

Minimum documentation searched (classification system followed by classification symbols)

A61K51/10; A61K31/198; A61K31/341; A61K31/585; A61K33/24; A61K39/395; A61K45/00; A61K47/22; A61K47/69; A61P13/12; A61P35/00; A61P39/04; A61P43/00; C07K16/18; C07K16/46

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-513695 A (IMMUNOMEDICS, INC.) 27 April 2006 (2006-04-27) claims 3-4, 12, 32, 48, examples 2-3, 5-6, 8 | 1-17 |
| Y | JP 2017-536340 A (IMMUNOMEDICS, INC.) 07 December 2017 (2017-12-07) paragraph [0012] | 1-17 |
| Y | JAGGI, J. S. et al. Efforts to control the errant products of a targeted in vivo generator. Cancer Research. 2005, vol. 65, issue 11, pp. 4888-4895, ISSN:0008-5472. abstract, fig. 2-3, 5, table 1-3 | 1-17 |
| Y | CN 111282064 A (NANFANG HOSPITAL) 16 June 2020 (2020-06-16) paragraph [0004] | 1-17 |
| Y | JP 2018-002667 A (KANEKA CORP) 11 January 2018 (2018-01-11) paragraph [0072] | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
**PCT/JP2022/018414**

**C. DOCUMENTS CONSIDERED TO BE RELEVANT**

| Category* | Citation of document, with indication, where appropriate, of the relevant passages | Relevant to claim No. |
|---|---|---|
| Y | WO 2019/203191 A1 (NIHON MEDI-PHYSICS CO., LTD.) 24 October 2019 (2019-10-24) claims 1, 6, paragraphs [0052], [0059], examples 4-5 | 11-13 |

Form PCT/ISA/210 (second sheet) (January 2015)

**INTERNATIONAL SEARCH REPORT**

International application No.
**PCT/JP2022/018414**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/018414**

| Patent document cited in search report | Publication date (day/month/year) | Patent family member(s) | Publication date (day/month/year) |
|---|---|---|---|
| JP 2006-513695 A | 27 April 2006 | US 2005/0014207 A1<br>claims 3-4, 12, 32, 48, examples 2-3, 5-6, 8<br>WO 2003/106495 A2<br>EP 1519958 A2<br>CN 1675245 A<br>KR 10-1143035 B1 | |
| JP 2017-536340 A | 07 December 2017 | US 2016/0095939 A1<br>paragraph [0012]<br>WO 2016/057398 A1<br>EP 3204018 A1<br>CN 106999517 A | |
| CN 111282064 A | 16 June 2020 | (Family: none) | |
| JP 2018-002667 A | 11 January 2018 | US 2019/0117731 A1<br>paragraph [0123]<br>EP 3479840 A1 | |
| WO 2019/203191 A1 | 24 October 2019 | US 2021/0170058 A1<br>claims 1, 6, paragraphs [0098], [0113], examples 4-5<br>EP 3783016 A1<br>CN 112041337 A<br>KR 10-2020-0143366 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H7203974 A
- JP H115749 A
- WO 2013157102 A
- WO 2013157105 A
- WO 2005028021 A
- JP 2007529459 A
- WO 2016186206 A
- WO 2017217347 A
- WO 2018230257 A
- WO 2003080655 A
- WO 2011118699 A
- JP 2021072206 A


### Non-patent literature cited in the description


- *Japanese Journal of Cancer Research,* 1996, vol. 87, 977-984
- *Japanese Journal of Clinical Medicine,* 2006, vol. 64 (1), 274-278
- *Japanese Journal of Cancer Research,* 1999, vol. 90, 1179-1186
- *Cancer Res.,* 01 June 2005, vol. 65 (11), 4888-95
- **BERNHARD et al.** DOTAGA-Anhydride: A Valuable Building Block for the Preparation of DOTA-Like Chelating Agents. *Chem. Eur. J.,* 2012, vol. 18, 7834-7841